# EUROPEAN PATENT SPECIFICATION

(11) **EP 1 490 489 B1**
(45) Date of publication and mention of the grant of the patent: **02.06.2010**
(21) Application number: 03707900.1
(22) Date of filing: 12.03.2003
(51) Int. Cl.: C12N 15/11, C12Q 1/68

(54) **METHODS AND MEANS FOR MONITORING AND MODULATING GENE SILENCING**
VERFAHREN UND MITTEL ZUR ÜBERWACHUNG UND MODULATION DES GEN-SILENCING
METHODES ET MOYENS DE SURVEILLANCE ET DE MODULATION DU BLOCAGE D'UN GENE

(30) Priority: 14.03.2002 US 363852 P
(43) Date of publication of application: 29.12.2004
(73) Proprietor: Commonwealth Scientific and Industrial Research Organisation, Campbell, ACT 2612 (AU)
(72) Inventor: WATERHOUSE, Peter, O'Connor, Australian Capital Territory 2602 (AU); WESLEY, Susan, Kaleen, Australian Capital Territory 2617 (AU); HELLIWELL, Chris, O'Connor, Australian Capital Territory 2602 (AU)
(74) Representative: Almond-Martin, Carol
(86) International application number: PCT/AU2003/000293
(87) International publication number: WO 2003/076620

(56) References cited:
- WO-A-02/10365
- WO-A-93/23551
- WESLEY S VARSHA ET AL: "Construct design for efficient, effective and high-throughput gene silencing in plants" PLANT JOURNAL, BLACKWELL SCIENTIFIC PUBLICATIONS, OXFORD, GB, vol. 27, no. 6, September 2001 (2001-09), pages 581-590, XP002187670 ISSN: 0960-7412
- HELLIWELL C A ET AL: "HIGH-THROUGHPUT VECTORS FOR EFFICIENT GENE SILENCING IN PLANTS" FUNCTIONAL PLANT BIOLOGY, CSIRO,, AU, vol. 29, no. 10, 2002, pages 1217-1225, XP009063510 ISSN: 1445-4408
- ELBASHIR S.M. ET AL.: 'Analysis of gene function in somatic mammalian cells using small interfering RNAs' METHODS vol. 26, February 2002, pages 199 - 213, XP002251055

## Description

### Field of the invention.

The present invention relates to methods of altering the expression of genes in eukaryotic organisms, such as plants, but also animals such as nematodes, insects and arthropods, mammals including humans, or yeasts, fungi or molds, using dsRNA capable of altering the expression of target genes, or genes encoding such dsRNA. Also provided are non-human eukaryotic organisms comprising such dsRNA or genes encoding it.

In a first aspect, the invention provides methods and means for monitoring the silencing of a target gene in a eukaryotic cell by measuring the degree of silencing of a second gene wherein the silencing of the target gene and of the second gene is obtained through the action of a single initial dsRNA molecule provided to the eukaryotic cell.

In a second aspect, the invention provides methods and means for modulating the degree of silencing of a target gene in a eukaryotic cell, by including into the single initial dsRNA molecule provided to the eukaryotic cell in addition to the target dsRNA inducing the silencing of the target gene, an amount of ds RNA sequences unrelated to the target dsRNA, in a proportion reflecting the desired modulation of the degree of silencing of the target gene.

### Background art.

Until recently, two predominant methods for the modulation of gene expression in eukaryotic organisms were known, which are referred to in the art as "antisense" downregulation or "sense" downregulation.

Recent work has demonstrated that the silencing efficiency could be greatly improved both on quantitative and qualitative level using chimeric constructs encoding RNA capable of forming a double stranded RNA by basepairing between the antisense and sense RNA nucleotide sequences respectively complementary and homologous to the target sequences. Such double stranded RNA (dsRNA) is also referred to as hairpin RNA (hpRNA) or interfering RNA (RNAi).

Down-regulating the expression of target nucleotide sequence using dsRNA has been described as a convenient analysis method for the elucidation of the function of nucleic acids the role of which is unknown (see e.g. WO99/53050 or WO00/01846). dsRNA mediated gene silencing can also be used to modulate the expression of one or more target genes in an organism to obtain a modified organism with a desired phenotype or trait (WO99/53050).

It would be helpful for the above mentioned applications to have a way for easy monitoring the quantitative and qualitative down-regulation of the target nucleic acids. This is particularly true where determination of the down-regulation of the expression of the target gene through the analysis of the phenotype caused by the down regulation is labor-intensive, cost-intensive, time-consuming, requires the use of specialized analysis methods, etc.. When dsRNA mediated gene silencing is used for function identification/validation of unknown genes, where by definition the phenotype to look for is not known, it may be imperative to have an alternative method for monitoring the efficiency of the silencing.

Further, it would be convenient to be able to modulate the degree of silencing of a particular target gene or genes in an organism e.g. by generating a population of organisms exhibiting a large spectrum in the individual degrees of dsRNA mediated gene silencing and identifying the organisms with the desired degree of silencing.

Fire *et al*., 1998 describe specific genetic interference by experimental introduction of double-stranded RNA in *Caenorhabditis elegans.*

WO 99/32619 provides a process of introducing an RNA into a living cell to inhibit gene expression of a target gene in that cell. The process may be practiced ex vivo or in vivo. The RNA has a region with double-stranded structure. Inhibition is sequence-specific in that the nucleotide sequences of the duplex region of the RNA and or a portion of the target gene are identical.

Waterhouse *et al.* 1998 describe that virus resistance and gene silencing in plants can be induced by simultaneous expression of sense and anti-sense RNA. The sense and antisense RNA may be located in one transcript that has self-complementarity.

Hamilton *et al.* 1998 describes that a transgene with repeated DNA, *i.e*., inverted copies of its 5' untranslated region, causes high frequency, post-transcriptional suppression of ACC-oxidase expression in tomato.

WO 98/53083 describes constructs and methods for enhancing the inhibition of a target gene within an organism which involve inserting into the gene silencing vector an inverted repeat sequence of all or part of a polynucleotide region within the vector.

WO 99/53050 provides methods and means for reducing the phenotypic expression of a nucleic acid of interest in eukaryotic cells, particularly in plant cells, by introducing chimeric genes encoding sense and antisense RNA molecules directed towards the target nucleic acid. These molecules are capable of forming a double stranded RNA region by base-pairing between the regions with the sense and antisense nucleotide sequence or by introducing the RNA molecules themselves. Preferably, the RNA molecules comprise simultaneously both sense and antisense nucleotide sequences.

WO 99/49029 relates generally to a method of modifying gene expression and to synthetic genes for modifying endogenous gene expression in a cell, tissue or organ of a transgenic organism, in particular to a transgenic animal or plant. Synthetic genes and genetic constructs, capable of forming a dsRNA which are capable of repressing, delaying or otherwise reducing the expression of an endogenous gene or a target gene in an organism when introduced thereto are also provided.

WO 99/61631 relates to methods to alter the expression of a target gene in a plant using sense and antisense RNA fragments of the gene. The sense and antisense RNA fragments are capable of pairing and forming a double-stranded RNA molecule, thereby altering the expression of the gene. The present invention also relates to plants, their progeny and seeds thereof obtained using these methods.

WO 00/01846 provides a method of identifying DNA responsible for conferring a particular phenotype in a cell which method comprises a) constructing a cDNA or genomic library of the DNA of the cell in a suitable vector in an orientation relative to (a) promoter(s) capable of initiating transcription of the cDNA or DNA to double stranded (ds) RNA upon binding of an appropriate transcription factor to the promoter(s); b) introducing the library into one or more of cells comprising the transcription factor, and c) identifying and isolating a particular phenotype of a cell comprising the library and identifying the DNA or cDNA fragment from the library responsible for conferring the phenotype. Using this technique, it is also possible to assign function to a known DNA sequence by a) identifying homologues of the DNA sequence in a cell, b) isolating the relevant DNA homologus(s) or a fragment thereof from the cell, c) cloning the homologue or fragment thereof into an appropriate vector in an orientation relative to a suitable promoter capable of initiating transcription of dsRNA from said DNA homologue or fragment upon binding of an appropriate transcription factor to the promoter and d) introducing the vector into the cell from step a) comprising the transcription factor.

WO 00/44914 also describes composition and methods for *in vivo* and *in vitro* attenuation of gene expression using double stranded RNA, particularly in zebrafish.

WO 00/49035 discloses a method for silencing the expression of an endogenous gene in a cell, the method involving overexpressing in the cell a nucleic acid molecule of the endogenous gene and an antisense molecule including a nucleic acid molecule complementary to the nucleic acid molecule of the endogenous gene, wherein the overexpression of the nucleic acid molecule of the endogenous gene and the antisense molecule in the cell silences the expression of the endogenous gene.

Smith et al., 2000 as well as WO 99/53050 described that intron containing dsRNA further increased the efficiency of silencing. Intron containing hairpin RNA is often also referred to as ihpRNA.

As illustrated by the above mentioned references, dsRNA mediated gene silencing is a phenomenon which occurs in a wide range of eukaryotic organisms, including plants, yeasts or fungi, insects, arthropods and vertebrate animals, including mammals.

WO 93/23551 describes a process for the inhibition of two or more target genes which comprises introducing into the plant a single control gene which has distinct DNA regions homologous to each of the target genes and a promoter operative in plants adapted to transcribe from such distinct regions either antisense or sense RNA that inhibits expression of each of the target genes.

WO 99/49029 describes a method for simultaneously targeting the expression of several target genes which are co-expressed in a particular cell, for example by using a dispersed nucleic acid molecule or foreign nucleic acid molecule which comprises nucleotide sequences which are substantially identical to each of said co-expressed target genes.

WO02/10365 provides a method for gene suppression in eukaryotes by transformation with a recombinant construct containing a promoter, at least one antisense and/or sense nucleotide sequence for the gene(s) to be suppressed, wherein the nucleus-to-cytoplasm transport of the transcription products of the construct is inhibited. In one embodiment, nucleus-to-cytoplasm transport is inhibited by the absence of a normal 3' UTR. The construct can optionally include at least one self-cleaving ribozyme. The construct can also optionally include sense and/or antisense sequences to multiple genes that are to be simultaneously down-regulated using a single promoter. Also disclosed are vectors, plants, animals, seeds, gametes and embryos containing the recombinant construct.

Elbashir et al., 2002, METHODS Vol 26, pages 199-213 reviews the design strategy for siRNAs and several silencing protocols, including double knockdown in HeLa SS6 cells of lamin A/C and NuMA.

Wesley et al. 2001 (The Plant Journal 27 (6), 581-590) describe the silencing vectors pHANNIAL and pHELLSGATE used in the present application.

However, none of the above mentioned prior art references have addressed the problem of monitoring the degree of silencing of a target gene with a phenotype that is difficult or practically impossible to measure. The prior art also remains defective in providing a method for modulating or fine-tuning the degree of silencing of a specific target gene or genes using dsRNA.

These and other problems have been solved as described hereinafter in the different embodiments and claims.

### SUMMARY OF THE INVENTION.

The invention provides a method for monitoring the reduction of the expression of a target gene in a cell of a eukaryotic organism, such as a plant, animal, yeast, fungus or mold, comprising the steps of
a) providing the cell of said eukaryotic organism with a dsRNA comprising a first region, a second region, a third region and a fourth region wherein
   i) the first region comprises a nucleotide sequence of at least 19 consecutive nucleotides having at least 94% sequence identity to 19 consecutive nucleotides from a sense nucleotide region of said target gene;
   ii) the second region comprises a nucleotide sequence of at least 19 consecutive nucleotides having at least 94% sequence identity to a nucleotide sequence complementary to 19 consecutive nucleotides from said sense nucleotide region of the target gene ;
   iii) the first region and the second region are capable of forming a double stranded RNA region:
   iv) the third region comprises a nucleotide sequence of at least 19 consecutive nucleotides having at least 94% sequence identity to 19 consecutive nucleotides from a sense nucleotide region of a second gene, such as an endogenous gene or a transgene, stably integrated into the genome of the eukaryotic cell and which is different from the target gene;
   v) the fourth region comprising a nucleotide sequence of at least 19 consecutive nucleotides having at least 94% sequence identity to the complement of the 19 consecutive nucleotides from the sense nucleotide region of the second gene;
   vi) the third and fourth region being capable of forming a double stranded RNA region; and
b) monitoring the reduction of the expression of the target gene by analyzing the reduction in expression of said second gene,
wherein the eukaryotic organism is a plant, a yeast, a fungus, a mold, or an animal with the proviso that when said organism is an animal, the cell is not a cell in a human or animal body.

The dsRNA may be transcribed from a chimeric gene comprised within cells of the eukaryotic organism, the chimeric gene comprising:
c) a promoter region which functions in the eukaryotic cell; operably linked to
d) a DNA region which when transcribed yields the dsRNA molecule; and
e) a transcription termination and polyadenylation region that functions in the cells of eukaryotic organism.

It is also an object of the invention to provide an RNA molecule as described as well as the use of such an RNA molecule for measuring the reduction of expression of a target gene.

It is another object of the invention to provide a DNA molecule for measuring the reduction of expression of a target gene in a eukaryotic cell comprising
a) a promoter region which functions in the eukaryotic cell; operably linked to
b) a DNA region which when transcribed yields a dsRNA molecule, wherein the dsRNA comprises a first, second, third and fourth region;
   i) the first region comprising a nucleotide sequence of at least 19 consecutive nucleotides having at least 94% sequence identity to 19 consecutive nucleotides from a sense nucleotide region of the target gene;
   ii) the second region comprising a nucleotide sequence of at least 19 consecutive nucleotides having at least 94% sequence identity to a nucleotide sequence complementary to 19 consecutive nucleotides from the sense nucleotide region of the target gene ;
   iii) the first region and said second region being capable of forming a double stranded RNA region:
   iv) the third region comprising a nucleotide sequence of at least 19 consecutive nucleotides having at least 94% sequence identity to 19 consecutive nucleotides from a sense nucleotide, region of a second gene present in the eukaryotic cell and which is different from the target gene;
   v) the fourth region comprising a nucleotide sequence of at least 19 consecutive nucleotides having at least 94% sequence identity to the complement of the 19 consecutive nucleotides from the sense nucleotide region of the second gene;
   vi) the third and fourth region being capable of forming a double stranded RNA region; wherein the double stranded RNA regions formed between the first and second region and the double stranded RNA region formed between the third and foruth region are about equal in size; and
c) a transcription termination and polyadenylation region that functions in cells of the eukaryotic organism, wherein said second gene is an endogenous gene of the eukaryotic organism or a transgene stably integrated into the genome of cells of the eukaryotic organism, as well as the use of such a DNA molecule to measure the expression of a target gene by measuring the reduction in expression of a second gene.

The invention also provides non-human eukaryotic organisms comprising an RNA molecule or a DNA molecule as herein described.

Also provided by the invention is a method for identifying, within a population of dsRNA-mediated gene-silenced eukaryotic organisms, those organisms with the desired degree of silencing of a target gene comprising:
a) providing the eukaryotic cells of the eukaryotic organisms with a dsRNA comprising a first region, a second region, a third region and a fourth region
   i) the first region comprising a nucleotide sequence of at least 19 consecutive nucleotides having at least 94% sequence identity to 19 consecutive nucleotides from a sense nucleotide region of the target gene;
   ii) the second region comprising a nucleotide sequence of at least 19 consecutive nucleotides having at least 94% sequence identity to a nucleotide sequence complementary to 19 consecutive nucleotides from the sense nucleotide region of the target gene ;
   iii) the first region and the second region being capable of forming a double stranded RNA region:
   iv) the third region comprising a nucleotide sequence of at least 19 consecutive nucleotides having at least 94% sequence identity to 19 consecutive nucleotides from a sense nucleotide region of a second gene present in the eukaryotic cells and which is different from the target gene;
   v) the fourth region comprising a nucleotide sequence of at least 19 consecutive nucleotides having at least 94% sequence identity to the complement of the 19 consecutive nucleotides from the sense nucleotide region of the second gene;
   vi) the third and fourth region being capable of forming a double stranded RNA region; and
b) identifying the organism with the desired degree of silencing of the target gene, by selecting those organisms with the desired degree of silencing of the second gene wherein the eukaryotic organism is a plant, a yeast, a fungus, a mold, or an animal, with the proviso that when said organism is an animal, the cell is not a cell in a human or animal body.

### BRIEF DESCRIPTION OF THE FIGURES

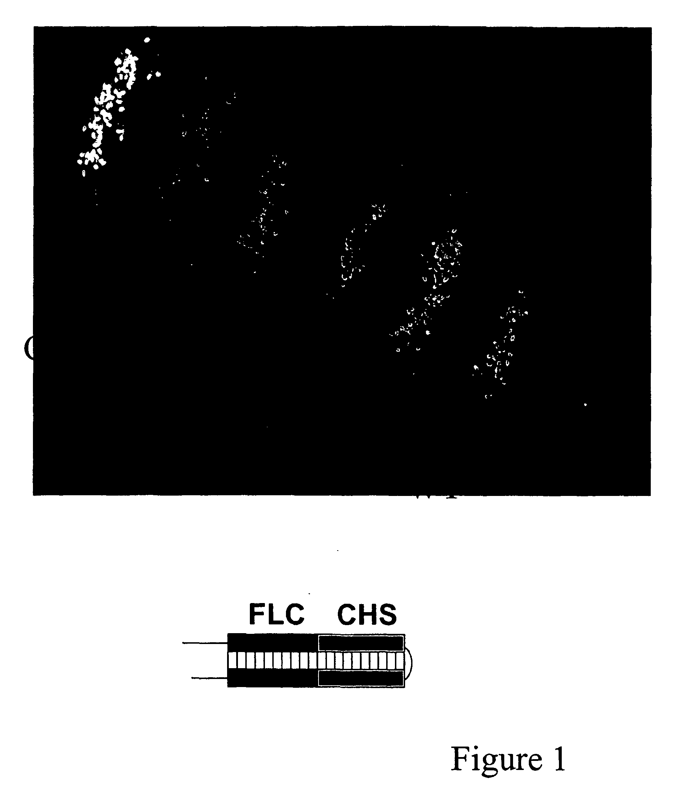

Figure 1 shows seeds from T1 plants transformed with the FLC/CHS construct, fluorescing under UV light, compared to seeds from C24 wildtype (C24), a C24 wildtype containing a chimeric GUS gene under control of a CaMV35S promoter (GUS) and a homozygous CHS silenced line (CHS). Numbers indicate the days to flowering of the transgenic plant cell lines.

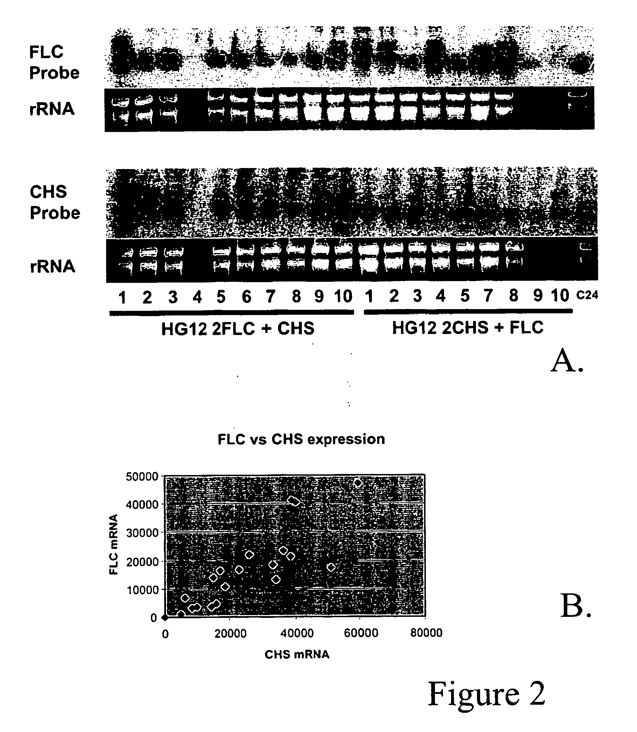

Figure 2 shows a Northern blot analysis of RNA prepared from transgenic plant lines, comprising dsRNA encoding genes targeted towards both FLC and CHS, probed with an FLC probe (upper panel) and a CHS probe (lower panel) (Fig 2A). Fig 2B is a graphic representation of the the amount of mRNA detected from FLC expression plotted against the amount of mRNA detected from CHS expression.

### DESCRIPTION OF DETAILED EMBODIMENTS

The invention is based on the unexpected observation that when a eukaryotic cell, such as a plant cell, comprises a double stranded RNA (dsRNA) wherein the dsRNA comprises simultaneously complementary antisense and sense regions for at least two target genes, a correspondence exists between the degree of silencing of expression of all of the targeted genes. The correspondence further depends upon the relative size of the antisense and sense regions designed to reduce the expression of the different target genes.

This correspondence may conveniently be used to monitor silencing of genes, the expression of which results in a phenotype which is not straightforward to monitor. This may be accomplished by linking the complementary sense and antisense regions suitable to reduce or silence the expression of such a gene, to complementary sense and antisense regions suitable for reducing or silencing the expression of a gene whose expression results in a phenotype which can be monitored in a straightforward way.

Thus, in one embodiment of the invention, a method is provided for monitoring the reduction or silencing of the expression of a target gene in a eukaryotic cell, comprising the steps of
- Providing the eukaryotic cell with a dsRNA comprising a first region, a second region, a third region and a fourth region, wherein
   - the first region comprises a nucleotide sequence of at least 19 consecutive nucleotides having at least 94% sequence identity to 19 consecutive nucleotides from the sense nucleotide region of the target gene;
   - the second region comprises a nucleotide sequence of at least 19 consecutive nucleotides having at least 94% sequence identity to a nucleotide sequence complementary to 19 consecutive nucleotides from the sense nucleotide region of the target gene ;
   - the first region and second region are capable of forming a double stranded RNA region, preferably over the entire length of the first and second region, and at least over the length of the mentioned 19 nucleotides ;
   - the third region comprises a nucleotide sequence of at least 19 consecutive nucleotides having at least 94% sequence identity to 19 consecutive nucleotides from the sense nucleotide region of a second gene present in the eukaryotic cell and which is different from the target gene;
   - the fourth region comprises a nucleotide sequence of at least 19 consecutive nucleotides having at least 94% sequence identity to the complement of 19 consecutive nucleotides from the sense nucleotide region of the same second gene;
   - the third and fourth region are capable of forming a double stranded RNA region, preferably over the entire length of the first and second region, and at least over the length of the mentioned 19 nucleotides; and
- monitoring the expression of the degree of silencing of the target gene, by analyzing the reduction in expression of the second gene.

Preferably, the first and second region are capable of forming a double stranded RNA region at the same time as when the third and fourth region are forming a double stranded RNA.

The second gene different from the target gene is used as a reporter gene to monitor the degree of silencing of the target gene and whenever reference is made herein to a reporter gene, it is understood that this term is used to refer to a second gene present in the eukaryotic cell and which is different from the target gene.

For the purposes of the current invention, it is equal whether that second gene is an endogene, normally present in the eukaryotic cell, or a transgene, which has been introduced into the eukaryotic cell by human intervention at some point in history, preferably integrated into the genome of the eukaryotic cell in a stable manner.

Preferably, the reporter gene should have a phenotype, the analysis of which is more straightforward than the analysis of the target gene or genes, either in terms of requirements of costs, expertise, time, labour, used apparatuses, etc.

Also preferably, the silencing of the reporter gene should not have a negative influence on the viability of the host cell or host organism, although the absence of negative influence may be dependent on particular conditions.

Conveniently, silencing the expression of the reporter gene may result in a visible phenotype, although the visibility of the phenotype may again be conditional.

Examples of reporter genes suitable for application of the methods of the invention in plant cells and plants include, but are not limited to Chalcone synthase gene (CHS, where down-regulation of the expression results in accumulation of UV-fluorescent compounds in the seed coat color); phytoene desaturase gene (PDS, where down-regulation of the gene expression results in photobleaching), flower locus C (FLC, where down-regulation of the gene expression results in early flowering), ethylene insensitivity gene 2 (EIN2, where down-regulation of the gene results in plants which are insensitive to ethylene, and will grow on media containing 1-aminocyclopropane-1-carboxylic acid(AAC)), visual marker gene such as seed coat color genes (e.g. R-gene in corn), plant-expressible GUS or GFP genes, phytochrome B and the like.

Examples of reporter genes suitable for application of the methods of the invention in animal cells and animals include but are not limited to GUS or GFP genes operably linked to expression regions suitable for animal cells but also genes such as *unc* in *Caenorhabditis elegans,* the silencing of which causes a characteristic twisting pattern in the nematodes, and the like.

Examples of reporter genes suitable for application of the methods of the invention in fungal cells include but are not limited to GUS or GFP genes operably linked to expression regions suitable for fungus cells but also genes the silencing of which causes auxotrophic growth (such as, e.g., trpC), a phenotype which can be easily screened on minimal media. It goes without saying that in these cases, a master copy of the library of silenced fungal cells needs to be maintained under conditions allowing growth, e.g., in the presence of the required nutrient compound.

Preferably, the reporter gene should be expressed at least under conditions where the target gene is expressed.

The 19 nucleotides which are at least 94% identical or complementary to a nucleotide sequence of 19 consecutive nucleotides of the target gene and/or the reporter gene may be comprised within a larger RNA molecule. Such an RNA molecule will have a nucleotide sequence varying between as little as 19 bp to a length equal to the size of the target gene with a varying overall degree of sequence identity.

For the purpose of this invention, the "sequence identity" of two related nucleotide or amino acid sequences, expressed as a percentage, refers to the number of positions in the two optimally aligned sequences which have identical residues (x100) divided by the number of positions compared. A gap, *i.e.,* a position in an alignment where a residue is present in one sequence but not in the other is regarded as a position with non-identical residues. The alignment of the two sequences is performed by the Needleman and Wunsch algorithm (Needleman and Wunsch 1970) The computer-assisted sequence alignment above, can be conveniently performed using standard software program such as GAP which is part of the Wisconsin Package Version 10.1 (Genetics Computer Group, Madision, Wisconsin, USA) using the default scoring matrix with a gap creation penalty of 50 and a gap extension penalty of 3. Sequences are indicated as "essentially similar" when such sequence have a sequence identity of at least about 75%, particularly at least about 80 %, more particularly at least about 85%, quite particularly about 90%, especially about 95%, more especially about 100%, quite especially are identical. It is clear than when RNA sequences are the to be essentially similar or have a certain degree of sequence identity with DNA sequences, thymine (T) in the DNA sequence is considered equal to uracil (U) in the RNA sequence. Thus when it is stated in this application that a sequence of 19 consecutive nucleotides has a 94% sequence identity to a sequence of 19 nucleotides, this means that at least 18 of the 19 nucleotides of the first sequence are identical to 18 of the 19 nucleotides of the second sequence.

The mentioned sense or antisense nucleotide regions may thus be about 50 nt, 100nt, 200 nt, 300nt, 500nt, 1000 nt, 2000 nt or even about 5000 nt or larger in length, each having an overall sequence identity of respectively about 40 %, 50%, 60 %, 70%, 80%, 90 % or 100%. The longer the sequence, the less stringent the requirement for the overall sequence identity.

The first, second, third and fourth region each may be separated by a spacer region having a nucleotide sequence which is unrelated to the nucleotide sequence of either the target or the reporter gene.

For the purpose of the invention, although the regions are named consecutively , the order of the dsRNA regions in the dsRNA molecule is not important. In other words, it does not matter whether, e.g., the first or second region or alternatively the third or fourth region are located at the 5' or 3' end of the RNA molecule.

As used herein "comprising" is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, *i.e*., be embedded in a larger nucleic acid or protein. A chimeric gene comprising a DNA region which is functionally or structurally defined, may comprise additional DNA regions etc.

An dsRNA molecule comprising a first, second, third and fourth region as herein defined may thus additionally include, e.g., a fifth and sixth region having a nucleotide region of 19 nucleotides with at least 94% sequence identity or complementarity to, e.g., a target gene, which may the same target gene as the first target gene, or may be a different one.

In one embodiment of the invention, the dsRNA molecule may further comprise one or more regions having at least 94% sequence identity to regions of 19 consecutive nucleotides from the sense nucleotide of the target genes, different from the 19 consecutive nucleotides as defined in the first region, and one or more regions having at least 94% sequence identity to 19 consecutive nucleotides from the complement of the sense nucleotide of the target gene, different from the 19 consecutive nucleotides as defined in the second region, wherein these additional regions can basepair amongst themselves. Similarly, the dsRNA may also comprise additionally one or more regions having at least 94% sequence identity to regions of 19 consecutive nucleotides of a reporter gene different from the 19 nucleotides of the third region and one or more regions having at least 94% sequence identity to the complementary regions of 19 consecutive nucleotides of the reporter genes, wherein these additional regions are capable of base-pairing between themselves. Again, no particularly order of the regions is required, and these regions may be dispersed among each other. Thus, *e*.*g*., dsRNA regions directed towards silencing of the target gene may be alternated with dsRNA regions directed towards silencing of the reporter gene, provided of course that basepairing between complementary RNA regions is still possible.

Conveniently, the dsRNA as described may be introduced into the host cell by introduction and possible integration of a chimeric gene, transcription of which yields such a dsRNA. Thus the invention is also aimed at providing such a chimeric gene comprising
- a promoter or a promoter region which is capable of being expressed in cells of the eukaryotic organism of interest; operably linked to a DNA region which when transcribed yields a dsRNA molecule comprising a first region, a second region, a third region and a fourth region, wherein
   - the first region comprises a nucleotide sequence of at least 19 consecutive nucleotides having at least 94% sequence identity to 19 consecutive nucleotides from the sense nucleotide sequence of the target gene;
   - the second region comprises a nucleotide sequence of at least 19 consecutive nucleotides having at least 94% sequence identity to the complement of 19 consecutive nucleotides from the sense nucleotide sequence of the target gene ;
   - the first region and second region are capable of forming a double stranded RNA region, preferably over the entire length of the first and second region and at least between the mentioned 19 nucleotides of the first and second region;
   - the third region comprises a nucleotide sequence of at least 19 consecutive nucleotides having at least 94% sequence identity to 19 consecutive nucleotides from the sense nucleotide sequence of a second gene present in the eukaryotic cell and which is different from the target gene;
   - the fourth region comprises a nucleotide sequence of at least 19 consecutive nucleotides having at least 94% sequence identity to the complement of 19 consecutive nucleotides from the sense nucleotide sequence of the same second gene;
   - the third and fourth region are capable of forming a double stranded RNA region, preferably over the entire length of the first and second region, and at least over the length of the mentioned 19 nucleotides;
      and wherein the double stranded RNA regions formed between the first and second region and the double stranded RNA region formed between the third and fourth region are about equal in size; and
   - a transcription termination and polyadenylation region suitable for the eukaryotic cell of choice.

As used herein, the term "promoter" denotes any DNA which is recognized and bound (directly or indirectly) by a DNA-dependent RNA-polymerase during initiation of transcription. A promoter includes the transcription initiation site, and binding sites for transcription initiation factors and RNA polymerase, and can comprise various other sites (e.g., enhancers), at which gene expression regulatory proteins may bind.

The term "regulatory region", as used herein, means any DNA, that is involved in driving transcription and controlling (*i.e*., regulating) the timing and level of transcription of a given DNA sequence, such as a DNA coding for a protein or polypeptide. For example, a 5' regulatory region (or "promoter region") is a DNA sequence located upstream (*i.e*., 5') of a coding sequence and which comprises the promoter and the 5'-untranslated leader sequence. A 3' regulatory region is a DNA sequence located downstream (*i.e*., 3') of the coding sequence and which comprises suitable transcription termination (and/or regulation) signals, including one or more polyadenylation signals.

In one embodiment of the invention the promoter is a constitutive promoter. In another embodiment of the invention, the promoter activity is enhanced by external or internal stimuli (inducible promoter), such as but not limited to hormones, chemical compounds, mechanical impulses, abiotic or biotic stress conditions. The activity of the promoter may also regulated in a temporal or spatial manner (tissue-specific promoters; developmentally regulated promoters).

In a particular embodiment of the invention, the promoter is a plant-expressible promoter. As used herein, the term "plant-expressible promoter" means a DNA sequence that is capable of controlling (initiating) transcription in a plant cell.

This includes any promoter of plant origin, but also any promoter of non-plant origin which is capable of directing transcription in a plant cell, *i.e*., certain promoters of viral or bacterial origin such as the CaMV35S (Hapster et al., 1988), the subterranean clover virus promoter No 4 or No 7 (WO9606932), or T-DNA gene promoters but also tissue-specific or organ-specific promoters including but not limited to seed-specific promoters (e.g., WO89/03887), organ-primordia specific promoters (An et al., 1996), stem-specific promoters (Keller et al., 1988), leaf specific promoters (Hudspeth et al., 1989), mesophyl-specific promoters (such as the light-inducible Rubisco promoters), root-specific promoters (Keller et al.,1989), tuber-specific promoters (Keil et al., 1989), vascular tissue specific promoters ( Peleman et al., 1989 ), stamen-selective promoters ( WO 89/10396, WO 92/13956), dehiscence zone specific promoters (WO 97/13865) and the like.

In another particular embodiment of the invention, the promoter is a fungus-expressible promoter. As used herein, the term "fungus-expressible promoter" means a DNA sequence which is capable of controlling (initiating) transcription in a fungal cell such as but not limited to the *A. nidulans trpC* gene promoter, or the *S. cerevisiae* GAL4 promoter that is capable of controlling (initiating) transcription in a fungal cell.

In yet another particular embodiment of the invention, the promoter is a animal-expressible promoter. As used herein, the term "animal-expressible promoter" means a DNA sequence which is capable of controlling (initiating) transcription in an animal cell and including but not limited to SV40 late and early promoters, cytomegalovirus CMV-IE promoters, RSV-LTR promoter, SCSV promoter, SCBV promoter and the like.

The dsRNA molecules useful for the invention may also be produced by *in vitro* transcription. To this end, the promoter of the chimeric genes according to the invention may be a promoter recognized by a bacteriophage single subunit RNA polymerase, such as the promoters recognized by bacteriophage single subunit RNA polymerase such as the RNA polymerases derived from the E. coli phages T7, T3, ϕI, ϕII, W31, H, Y, A1, 122, cro, C21, C22, and C2; Pseudomonas putida phage gh-1; Salmonella typhimurium phage SP6; Serratia marcescens phage IV; Citrobacter phage ViIII and Klebsiella phage No.11 [Hausmann, Current Topics in Microbiology and Immunology, 75: 77-109 (1976); Korsten et al., J. Gen Virol. 43: 57-73 (1975); Dunn et al., Nature New Biology, 230: 94-96 (1971); Towle et al., J. Biol. Chem. 250: 1723-1733 (1975); Butler and Chamberlin, J. Biol. Chem., 257: 5772-5778 (1982)]. Examples of such promoters are a T3 RNA polymerase specific promoter and a T7 RNA polymerase specific promoter, respectively. A T3 promoter to be used as a first promoter in the CIG can be any promoter of the T3 genes as described by McGraw et al, Nucl. Acid Res. 13: 6753-6766 (1985). Alternatively, a T3 promoter may be a T7 promoter which is modified at nucleotide positions -10, - 11 and -12 in order to be recognized by T3 RNA polymerase [(Klement et al., J. Mol. Biol. 215, 21-29(1990)]. A preferred T3 promoter is the promoter having the "consensus" sequence for a T3 promoter, as described in US Patent 5,037,745. A T7 promoter which may be used according to the invention, in combination with T7 RNA polymerase, comprises a promoter of one of the T7 genes as described by Dunn and Studier, J. Mol. Biol. 166: 477-535 (1983). A preferred T7 promoter is the promoter having the "consensus" sequence for a T7 promoter, as described by Dunn and Studier (supra).

dsRNA can be produced in large amounts by contacting the acceptor vector DNA with the appropriate bacteriophage single subunit RNA polymerase under conditions well known to the skilled artisan. The so-produced dsRNA can then be used for delivery into cells prone to gene silencing, such as plant cells, fungal cells or animal cells. dsRNA may be introduced in animal cells via liposomes or other transfection agents (e.g. Clonfection transfection reagent or the CalPhos Mammalian transfection kit from ClonTech) and could be used for methods of treatment of animals, including humans, by silencing the appropriate target genes. dsRNA can be introduced into the cell in a number of different ways. For example, the dsRNA may be administered by microinjection, bombardment by particles covered by the dsRNA, soaking the cell or organims in a solution of the dsRNA, electroporation of cell membranes in the presence of dsRNA, liposome mediated delivery of dsRNA and transfection mediated by chemicals such as calcium phosphate, viral infection, transformation and the like. The dsRNA may be introduced along with components that enhance RNA uptake by the cell, stabilize the annealed strands, or otherwise increase inhibition of the target gene. In the case of a whole animal, the dsRNA is conveniently introduded by injection or perfusion into a cavity or interstitial space of an organims, or systemically via oral, topical, parenteral (including subcutaneous, intramuscular or intravenous administration), vaginal, rectal, intranasal, ophthalmic, or intraperitoneal administration. The dsRNA may also be administered via an implantable extended release device.

The chimeric genes according to the invention capable of producing a dsRNA may also be equipped with any prokaryotic promoter suitable for expression of dsRNA in a particular prokaryotic host. The prokaryotic host can be used as a source of dsRNA, e.g. by feeding it to an animal, such as a nematode or an insect, in which the silencing of the target gene is envisioned and monitored by reduction of the expression of the reporter gene. In this case, it will be clear that the target gene and reporter genes should be genes present in the cells of the target eukaryotic organism and not of the prokaryotic host organism. The dsRNA according to the invention or chimeric genes capable of yielding such dsRNA molecules, can be thus produced in one host organism. The dsRNA can be administered to another target organims (e.g. through feeding, orally administring, as a naked DNA or RNA molecule or encapsulated in a liposome, in a virus particle or attentuated virus particle, or on an inert particle etc.) and effect reduction of gene expression in the target gene or genes and reporter gene or genes in another organism. In this case, it will be clear that the target gene and reporter genes should be genes present in the cells of the target (eukaryotic) organism and not of the host organism.

Suitable transcription termination and polyadenylation regions include but are not limited to the SV40 polyadenylation signal, the HSV TK polyadenylation signal, the nopaline synthase gene terminator of *Agrobacterium tumefaciens,* the terminator of the CaMV 35S transcript, terminators of the subterranean stunt clover virus, the terminator of the *Aspergillus nidulans* trpC gene and the like.

The invention also aims at providing the dsRNA molecules, which may be obtained by transcription from these chimeric genes, and which are useful for the methods according to the invention.

It is another object of the invention to provide eukaryotic cells, and eukaryotic non-human organisms containing the dsRNA molecules of the invention, or containing the chimeric genes capable of producing the dsRNA molecules of the invention. In a preferred embodiment the chimeric genes are stably integrated in the genome of the cells of the eukaryotic organism.

In another preferred embodiment, the chimeric genes may be provided on a DNA molecule capable of autonomously replicating in the cells of the eukaryotic organism, such as e.g. viral vectors. The chimeric gene or the dsRNA may be also be provided transiently to the cells of the eukaryotic organism.

The dsRNA molecules according to this first aspect of the invention can also be used for identifying, within a population of dsRNA-mediated gene-silenced organisms, the ones with the desired degree of silencing of a target gene or genes. To this end, a population of dsRNA containing organisms is generated, wherein the dsRNA comprising a first region, a second region, a third region and a fourth region, wherein the first region comprises a nucleotide sequence of at least 19 consecutive nucleotides having at least 94% sequence identity to 19 consecutive nucleotides from the sense nucleotide sequence of the target gene. The second region comprises a nucleotide sequence of at least 19 consecutive nucleotides having at least 94% sequence identity to the complement of 19 consecutive nucleotides from the sense nucleotide sequence of the target gene. The third region comprises a nucleotide sequence of at least 19 consecutive nucleotides having at least 94% sequence identity to 19 consecutive nucleotides from the sense nucleotide sequence of a second gene present in the eukaryotic cell and which is different from the target gene. The fourth region comprises a nucleotide sequence of at least 19 consecutive nucleotides having at least 94% sequence identity to the complement of 19 consecutive nucleotides from the sense nucleotide sequence of the same second gene. The first and second and the third and fourth region are capable of forming a double stranded RNA region, preferably over the entire length of the first and second or third and fourth region, and at least between the mentioned 19 nucleotide stretches, and wherein the double stranded RNA regions formed between the first and second region and the double stranded RNA region formed between the third and fourth region are about equal in size. The population of dsRNA-containing organisms is then analyzed for the down-regulation of the expression of the reporter gene and dsRNA- containing organisms wherein the down-regulation of the expression of the reporter gene corresponds to the desired degree of down-regulation are selected and isolated.

The correspondence in the degree of silencing of two or more genes induced by the presence of dsRNA that comprises the double stranded complementary sense and antisense RNA regions for the two or more target genes, may also be used to modulate the degree of silencing of those target genes. The correspondence in the degree of silencing of two or more genes is dependent on the relative size of the double stranded complementary sense and antisense RNA regions. Consequently, the degree of silencing of one particular gene may be modulated by including into the same dsRNA molecule comprising complementary sense and antisense RNA regions, designed to silence the expression of a particular target gene or genes, an excess of unrelated complementary sequences, also capable of forming a double stranded RNA by base-pairing.

Without restricting the invention to a particular mode of action, it is thought that the enzyme in eukaryotic cells which is responsible for generating the small RNA molecules of about 21 nt from the dsRNA (such as DICER in *Drosophila*) may be saturated by including into the dsRNA an excess of dsRNA sequences (i.e, complementary RNA molecules), the sequence of which is unrelated to the nucleotide sequence of the target gene or genes to be silenced.

Thus in one embodiment of the invention, a method is provided for modulating the reduction of the expression of a target gene in a eukaryotic organism, comprising the steps of
- providing the eukaryotic cell with a dsRNA comprising a first region, a second region, a third region and a fourth region, wherein
   - the first region comprises a nucleotide sequence of at least 19 consecutive nucleotides having at least 94% sequence identity to 19 consecutive nucleotides from the sense nucleotide sequence of the target gene;
   - the second region comprises a nucleotide sequence of at least 19 consecutive nucleotides having at least 94% sequence identity to the complement of 19 consecutive nucleotides from the sense nucleotide sequence of the target gene ;
   - the first region and second region are capable of forming a double stranded RNA region preferably over the entire length of the first and second region, and at least between the mentioned stretches of 19 nucleotides;
   - the third region and fourth region comprise complementary nucleotide seqeunces unrelated to the nucleotide sequence of the target gene and are capable of forming a double stranded RNA. The size of the double stranded RNA capable of being formed by base-pairing between the third and fourth region, is preferably equal or larger than the size of the double stranded RNA capable of being formed by base-pairing between the first and the second region. The size of the third/fourth region may also be smaller than the size of the first/second region, depending on the desired degree of modulation of the silencing.

Preferably, the first and second region are capable of forming a double stranded RNA region at the same time as when the third and fourth region are forming a double stranded RNA. In addition, preferably, the target gene is an endogenous gene of the eukaryotic cell or a transgene, stably integrated into the genome of the eukaryotic cells.

As used herein, a sequence is said to be "unrelated" to a target gene, when the overall sequence identity between the unrelated sequence and the target sequence is less than 50%, particularly less than 45%, more particularly less than 35%. The unrelated sequence preferably should not have a nucleotide sequence having at least 94% sequence identity with a stretch of 19 consecutive nucleotides of the target gene or with the complement therof.

The natural variation in down regulation of the expression of a target gene occuring between different lines of a eukaryotic organism comprising the same dsRNA molecule will be shifted towards the lower end of the spectrum of gene silencing. This is due to inclusion of extra dsRNA nucleotide sequences unrelated to the target gene, which are operably linked to the dsRNA formed by the first and second RNA region.

The ratio of the size of the double stranded RNA capable of being formed by base-pairing between the third and fourth region to the size of the double stranded RNA capable of being formed by base-pairing between the first and the second region may vary from 0,1 to 20, preferably from 1 to 10. The greater the mentioned ratio, the more organisms in a population of eukaryotic organisms comprising that dsRNA will have a lower degree of gene-silencing *(i.e.,* a higher level of expression of the target gene).

The 19 nucleotides of the first or second RNA region which are at least 94% identical or complementary to a nucleotide sequence of 19 consecutive nucleotides of the target gene may be comprised within a larger RNA molecule. Such a longer RNA molecule will have a nucleotide sequence varying between as little as 19 bp to a length equal to the size of the target gene with a varying overall degree of sequence identity.

The mentioned sense or antisense nucleotide regions may thus be about 50 nt, 100nt, 200 nt, 300nt, 500nt, 1000 nt, 2000 nt or even about 5000 nt or larger in length, each having an overall sequence identity of respectively about 40 %, 50%, 60 %, 70%, 80%, 90 % or 100%. The longer the sequence, the less stringent the requirement for the overall sequence identity.

The first, second, third and fourth region each may be separated by a spacer region having a nucleotide sequence which is unrelated to the nucleotide sequence of either the target or the reporter gene.

Conveniently, the dsRNA molecules may be introduced into the eukaryotic cells or organisms by transcription from a chimeric gene comprising a promoter or promoter region capable of transcribing a DNA region, which when transcribed yields the dsRNA according to this aspect of the invention. It is understood that different embodiments concerning suitable promoters etc. mentioned in relation to the first aspect of the invention can also be applied here.

The invention also aims at providing dsRNA or chimeric genes capable of producing such dsRNA molecules according to the second aspect of the invention, as well as non-human eukaryotic organisms comprising such dsRNA or chimeric genes.

The inverted repeat part of the chimeric genes of the invention can be conventionally constructed by recombinational cloning, using the means and methods described in US patent applications 60/244067 and 60/333743 or WO02/059294 Vectors according to US patent applications 60/244067 or US 60/333743 may be modified to include a third and fourth region capable of forming a double stranded RNA gene, wherein the third and fourth region are capable of downregulating the expression of a marker gene such as EIN2, PHYB, FLC and CHS.

The chimeric genes encoding dsRNA according to the invention preferably comprise an intron, preferably in the region between the second and third region, in order to increase the efficiency (as described in WO99/53050). As used herein, an "intron" or intervening sequence is used to refer to a DNA region within a larger transcribed region, which is transcribed in the nucleus to yield an RNA region which is part of a larger RNA, however, said RNA corresponding to the intron sequence is removed from the larger RNA when transferred to the cytoplasm. The corresponding RNA is also referred to as an intron or intervening sequence. Intron sequences are flanked by splicing sites, and synthetic introns may be made by joining appropriate splice sites to any sequence, having an appropriate branching point. Examples of introns include the pdk2 intron, catalase intron from Castor bean, Delta 12 desaturase intron from cotton, Delta 12 desaturase intron from *Arabidopsis,* ubiquitin intron from maize or the intron from SV40.

In one embodiment of the invention, combining the first and second aspect of the invention, a dsRNA molecule may be provided to a eukaryotic cell for monitoring the down regulation of the expression of a target gene by analyzing the down regulation of a second or reporter gene, as described herein. In such an embodiment, the ratio between the size of the dsRNA having sequence to the target gene or its complement and the size of the dsRNA having sequence to the second gene or its complement is between 2 and 10. It is expected that in this way, by identifying the organisms wherein the expression of second gene is effectively downregulated, a population of organisms wherein the expression of the target gene is severely downregulated will be identified.

The methods and means described herein, can be applied to any eukaryotic organism in which gene-silencing takes place, including but not limited to plants (such as corn, cotton, *Arabidopsis,* rice, vegetables, soybeans, tobacco, trees etc.) invertebrate animals (such as insects, shellfish, molluscs, crustaceans such as crabs, lobsters and prawns) vertebrate animals (fish, avian animals, mammals, humans), yeast and fungi amongst others.

The following non-limiting Examples describe method and means for monitoring dsRNA mediated silencing of the expression of a target gene through analysis of a second gene, as well as methods and means for modulating the degree of dsRNA mediated gene silencing in eukaryotic cells.

Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK. Other references for standard molecular biology techniques include Sambrook and Russell (2001) Molecular Cloning: A Laboratory Manual, Third Edition, Cold Spring Harbor Laboratory Press, NY, Volumes I and II of Brown (1998) Molecular Biology LabFax, Second Edition, Academic Press (UK). Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

Throughout the description and Examples, reference is made to the following sequences:
SEQ ID N° 1: oligonucleotide primer for PCR reaction of the CHS/FLC fragment.
SEQ ID N°2: oligonucleotide primer for PCR reaction of the CHS/FLC fragment.
SEQ ID N°3: oligonucleotide primer for PCR reaction of the CHS/FLC fragment.
SEQ ID N°4: oligonucleotide primer for PCR reaction of the CHS/FLC fragment.
SEQ ID N°5: attB1 recombination site.
SEQ ID N° 6: attB2 recombination site.
SEQ ID N°7: pHELLSGATE 8.

### EXAMPLES

### Example 1. An ihpRNA construct targeting two genes gives stoichiometric silencing.

### Construction of an ihpRNA construct targeting two genes.

A chimeric gene encoding an ihpRNA construct targeting two distinct genes was constructed comprising 300 nt of the Flower Locus C (*FLC*) gene (Genbank accession AF116527, bases 620-920) followed by 300 nt of the Chalcone synthase (*CHS*) gene (Genbank accession Y18603, bases 147-532) in sense orientation, and a nucleotide sequence complementary to the mentioned 300 nt of CHS followed by the complementary nucleotide sequence to the mentioned 300 nt of FLC.

The CHS/FLC insert was generated by amplifying a 300 bp fragment of FLC with the oligonucleotide primers having the following sequences:
- 5'CTCGAGTCTAGAGGAGCAGAAGCTGAGATGGAG 3' (SEQ ID N° 1) (primer 138)
- 5'CTGCAGGAATAAGGTACAAAGTTCATC 3' (SEQ ID N° 2) (primer 136) and cloning the resulting product into pGEM T-easy (Promega, Madison, WI).

The CHS fragment was amplified with the oligonucleotide primers having the following sequences:
- 5' CTGCAGGCACTGCTAACCCTGAGAAAC 3' (SEQ ID N° 3) (primer 133)
- 5' GGTACCTTGACTTGGGCTGGCCCCACT 3' (SEQ ID N° 4) (primer 143) and also cloned into pGEM T-easy.

A 300 bp fragment was excised from pGEM T-easy CHS containing the CHS fragment and inserted into the Pstl site of pGEM T-easy FLC. The resulting CHS/FLC chimeric insert was amplified using primers with the same gene-specific sequence as 138 and 143 but modified to comprise also the nucleotide sequence encoding the recombination sites attB1 (GGGGACAAGTTTGTACAAAAAAGCAGGCT; SEQ ID N° 5) and attB2 (GGGACCACTTTGTACAAGAAAGCTGGGT; SEQ ID N° 6), respectively at their 5' ends with F1 Taq DNA polymerase (Fisher Biotec, Subiaco, WA, Australia) using the manufaturer's protocol.

PCR products were precipitated by adding 3 volumes TE and two volumes 30% (w/v) PEG 3000, 30mM MgCl₂ and centrifuging at 13000 g for 15 minutes. Recombination reaction of PCR products with pDONR201 (Invitrogen, Groningen, The Netherlands) were carried out in a total volume of 10 µl with 2 µL BP clonase buffer (Invitrogen), 1-2 µL PCR product 150 ng plasmid vector and 2 µL BP clonase (Invitrogen). The reaction was incubated at room temperature (25°C) for 1 h to overnight. After the incubation, 1 µL proteinase K (2 µg/µL; Invitrogen) was added and incubated for 10 min at 37°C. 1-2 µL of the mix was used to transform E. coli DH5α, colonies were selected on the appropriate antibiotics. Clones were checked either by digestion of DNA minipreps or PCR.

Recombination reactions from pDONR201 clones to pHellsgate 8 were carried out in 10 µL total volume with 2 µL LR clonase buffer (Invitrogen), 2 µL pDONR201 clone (approximately 150 ng), 300 ng pHellsgate 8 and 2 µL LR clonase (Invitrogen). The reaction was incubated overnight at room temperature the proteinase treated and used to transform DH5α as for the BP clonase reaction.

pHELLSGATE 8 is a vector suitable for recombinational cloning in such a way that an inverted repeat of the insert DNA of interest is generated. The construction of pHELLSGATE 8, and its use for recombination cloning have been described in PCT application PCT/AU02/00073, US 60/264,067 or US priority application US 60/333,743. pHELLSGATE 8 comprises the following DNA elements encoding
- a right border sequence of *Agrobacterium tumefaciens* T-DNA region;
- a CaMV 35S promoter region;
- an *att*R1 recombination site;
- a ccdB selection marker gene from E. *coli;*
- an *att*R2 recombination site;
- a *pdk*2 (*Flavineria trinerva* pyruvate orthophosphate dikinase intron 2) intron sequence;
- a *att*R2 recombination site;
- a *ccd*B selection marker gene;
- an *att*R1 recombination site;
- a terminator region from *Agrobacterium tumefaciens* octopine synthase gene (ocs);
- a chimeric *npt*II gene flanked by a nopaline synthase gene (*nos*) promoter and nos terminator;
- a left border *Agrobacterium tumefaciens* T-DNA region;
- an origin of replication for E. coli and a streptomycine resistance gene.

The complete sequence of pHELLSGATE 8 is represented in SEQ ID N° 7.

Upon recombinational cloning of the CHS/FLC, PCR amplified insert as described above, a chimeric gene is thus generated comprising:
- a CaMV35S promoter region;
- a nucleotide sequence corresponding to bases 620-920 of *FLC;*
- a nucleotide sequence corresponding to bases 147-532 of C*HS*;
- a pdk2 intron
- a nucleotide sequence complementary to bases 147-532 of C*HS*;
- a nucleotide sequence complementary to bases 620-920 of *FLC;*
- an ocs terminator region.

This chimeric gene is located between *Agrobacterium* T-DNA border sequences, together with a plant-expressible selectable marker gene. The vector can thus be introduced into *Agrobacterium* comprising the required helper functions, and used to transform plant cells.

### Plant transformation.

Transformation of *Arabidopsis* C24 ecotype was via the floral dip method (Clough and Bent, 1998). Plants were selected on agar solidified MS media supplemented with 100 mg/l timentin and 50 mg/l kanamycin.

### Phenotypic analysis methods for plants wherein FLC or CHS genes are silenced.

T1 *FLC* ihpRNA plants were scored by transferring to MS plates and scoring days to flower or rosette leaves at flowering compared to C24 wild type plants and *flc* mutant lines. Plant lines wherein the FLC gene expression is reduced or eliminated flower earlier than wild type C24 ecotype. A block in anthocyanin biosynthesis by reducing expression of chalcone synthase gene (CHS) leads to the accumulation of malonyl-CoA, which increases fluorescence of seed observed under UV light.

All of the T1 plants transformed with the iph CHS/FLC construct flowered earlier than wild type C24 (36 days) and later than the transposon-tagged flc-13 line (17drays; Sheldon et al, 1999). The seeds from four ihp CHS/FLC plants that flowered at different times were tested for fluorescence (Figure 1) compared to wildtype C24, a 35S::GUS line and a homozygous ihpRNA-silenced CHS line (Wesley et al, 2001). The relative concentrations of the different intermediates in the chalcone synthase pathway were also determined by HPLC analysis.

The CHS ihpRNA line shows strong fluorescence reflecting the accumulation of malonyl-CoA in this line while the wildtype C24 and the 35S::GUS lines do not show fluorescence under UV light. The four ihp CHS/FLC lines examined all show some fluorescence with the earliest flowering lines showing the strongest fluorescence.

### Example 2. Modulation of the degree of silencing of CHS gene by dilution with extra non-target sequences in the stem of the iphRNA.

To test the influence of the inclusion of extra nucleotides, unrelated to the target DNA sequences, in the ihpRNA or dsRNA molecules on the degree of silencing of a target gene, a number of chimeric genes were generated each comprising 100 bp from the CHS nucleotide sequence (corresponding to Genbank accession Y18603) both in anti-sense and sense orientation (referred to as CHSₐₙₜᵢₛₑₙₛₑ and CHSsense respectively.)

Progressively more nucleotide sequences (from 100 bp to 900 bp; referred to as Extra-NNN-ntₛₑₙₛₑ or Extra-NNN-ntₐₙₜᵢₛₑₙₛₑ) unrelated to the CHS gene were inserted both in antisense and sense orientation. The unrelated nucleotide sequences were derived from the FLC gene (corresponding to Genbank accession Nr AF116527)

The different CHS/Extra nucleotide constructs were PCR amplified with attB1 and attB2 extended primers and introduced into pHELLSGATE 8 as described in Example 1. The resulting plasmids thus contain the following chimeric dsRNA genes:
1. <CaMV35S-CHSₛₑₙₛₑ-Extra_100_ntₛₑₙₛₑ-pdk2intron-Extra_100_ntₐₙₜᵢₛₑₙₛₑ-CHSₐₙₜᵢₛₑₙₛₐ-ocs terminator> or
2. <CaMV35S-CHSₛₒₙₛₑ-Extra_300_ntₛₑₙₛₑ-pdk2intron-Extra_300_ntₐₙₜᵢₛₑₙₛₑ-CHSₐₙₜᵢₛₑₙₛₒ-ocs terminator> or
3. <CaMV35S-CHSₛₑₙₛₑ-Extra_500_ntₛₑₙₛₑ-pdk2intron-Extra_500_ntₐₙₜᵢₛₒₙₛₐ-CHSₐₙₒₛₑₙₛₑ-ocs terminator> or
4. <CaMV35S-CHSₛₑₙₛₑ-Extra_900 _ntₛₑₙₛₑ-pdk2intron-Extra_900_ntₐₙₜᵢₛₑₙₛₑ-CHSₐₙₜiₛₑₙₛₒ-ocs terminator>

Transformed *Arabidopsis* plant lines comprising one of the four above mentioned chimeric genes were generated as described in Example 1, and fluorescence under UV light to monitor silencing of the CHS gene was scored as described in Example 1 for seeds of plants of each line. Independent transgenic lines were classified according to the degree of fluorescence. The results are summarized in Table 1.

**Table 1: Number of independent transgenic lines showing different classes of fluorescence.**

| | Fluorescence level | | | |
|---|---|---|---|---|
| | - | + | ++ | +++ |
| 100 nt CHS+ 100 extra nt | 12 | 0 | 20 | 0 |
| 100 nt CHS+ 300 extra nt | 11 | 18 | 11 | 1 |
| 100 nt CHS+ 500 extra nt | 5 | 7 | 13 | 0 |
| 100 nt CHS+ 900 extra nt | 18 | 8 | 3 | 0 |

From these results, it is clear that the higher the ratio of extra nucleotide sequences versus target sequences in the ihpRNA construct, the higher the proportion of transgenic plant lines obtained with a lower degree of fluorescence, *i*.*e*., with a lower degree of silencing of the expression of the CHS target gene.

Inclusion of extra, unrelated, sequences in ihpRNA or dsRNA effectively shifts the distribution of silencing in the population of transgenic lines towards the lower end of the spectrum of gene-silencing.

### Example 3. Northern analysis of transgenic plant lines comprising a chimeric gene encoding simultaneously dsRNA regions targeted towards FLC and CHS.

RNA was prepared from different *Arabidopsis* plant lines comprising two FLCCHS hairpin constructs. One construct was a modified Hellsgate12 vector (see WO02/059294) with a FLC hairpin next to the intron with a CHS fragment inserted in the attR sites. The other construct was a modified Hellsgate12 with a CHS hairpin next to the intron with an FLC fragment inserted into the attR sites. Two identical gels were run and blotted and probed with either FLC or CHS antisense RNA probes. The blots were RNase treated to ensure the signals were specific. The resulting autoradiogram is represented in Figure 2A. Estimates of the amount of hybridization for the different probes and lines are summarized in Table 2 and graphically represented in Fig 2B. There appears be a good linear direct relation between the amount of FLC and CHS signals in each line indicating that where one gene is silenced the other gene is also silenced at the same degree.

**Table 2. Estimates of hybridization signals in different plants using either FLC or CHS antisense probes.**

| Plant | FLC mRNA | CHS mRNA |
|---|---|---|
| HG12 2FLC + 2CHS #1 | 13867 | 15154 |
| HG 12 2FLC + 2CHS #2 | 21962 | 26467 |
| HG 12 2FLC + 2CHS #3 | 40434 | 40096 |
| HG12 2FLC + 2CHS#4 | 0 | 0 |
| HG 12 2FLC + 2CHS #5 | 41122 | 38987 |
| HG12 2FLC + 2CHS #6 | 16609 | 23091 |
| HG12 2FLC + 2CHS #7 | 18417 | 3357 |
| HG 12 2FLC + 2CHS #8 | 16463 | 17400 |
| HG 12 2FLC + 2CHS # 9 | 47210 | 59680 |
| HG12 2FLC + 2CHS #10 | 10792 | 18879 |
| HG12 2CHS + 2FLC #1 | 3399 | 9694 |
| HG12 2CHS + 2FLC #2 | 3692 | 14317 |
| HG12 2CHS + 2FLC #3 | 1725 | 51004 |
| HG12 2CHS + 2FLC #4 | 4620 | 16067 |
| HG12 2CHS + 2FLC #5 | 21826 | 38643 |
| HG12 2CHS + 2FLC #7 | 13288 | 34043 |
| HG12 2CHS + 2FLC #8 | 6716 | 5949 |
| HG12 2CHS + 2FLC #9 | 3027 | 8036 |
| HG12 2CHS + 2FLC #10 | 1021 | 4677 |
| C24 wildtype | 23376 | 36279 |

### References

An et al., 1996 The Plant Cell 8, 15-30
Butler and Chamberlin, 1982 J. Biol. Chem., 257: 5772-5778
Dunn and Studier 1983 J. Mol. Biol. 166: 477-535
Dunn et al. , 1971 Nature New Biology, 230: 94-96
Fire et al., 1998 Nature 391, 806-811
Hamilton et al. 1998 Plant J. 15: 737-746
Hapster et al., 1988 Mol. Gen. Genet. 212, 182-190
Hausmann, 1976 Current Topics in Microbiology and Immunology, 75: 77-109
Hudspeth et al., 1989 Plant Mol Biol 12: 579-589
Keil et al., 1989 EMBO J. 8: 1323-1330
Keller et al., 1988 EMBO J. 7: 3625-3633
Keller et al., 1989 Genes Devel. 3: 1639-1646
Klement et al., 1990 J. Mol. Biol. 215, 21-29
Korsten et al., 1975 J. Gen Virol. 43: 57-73
McGraw et al, 1985 Nucl. Acid Res. 13: 6753-6766
Needleman and Wunsch 1970
Peleman et al., 1989 Gene 84: 359-369
Smith et al., 2000 Nature 407: 319-320
Towle et al., J. Biol. Chem. 250: 1723-1733 (1975)
Waterhouse et al. 1998 Proc. Natl. Acad. Sci. USA 95: 13959-13964

## Claims

1. A method for monitoring the reduction of the expression of a target gene in a cell of a eukaryotic organism, said method comprising the steps of
a) providing the cell of said eukaryotic organism with a dsRNA comprising a first region, a second region, a third region and a fourth region
i) said first region comprising a nucleotide sequence of at least 19 consecutive nucleotides having at least 94% sequence identity to 19 consecutive nucleotides from a sense nucleotide region of said target gene;
ii) said second region comprising a nucleotide sequence of at least 19 consecutive nucleotides having at least 94% sequence identity to a nucleotide sequence complementary to 19 consecutive nucleotides from said sense nucleotide region of the target gene ;
iii) said first region and said second region being capable of forming a double stranded RNA region:
iv) said third region comprising a nucleotide sequence of at least 19 consecutive nucleotides having at least 94% sequence identity to 19 consecutive nucleotides from a sense nucleotide region of a second gene present in said eukaryotic cell and which is different from said target gene;
v) said fourth region comprising a nucleotide sequence of at least 19 consecutive nucleotides having at least 94% sequence identity to the complement of said 19 consecutive nucleotides from said sense nucleotide region of said second gene;
vi) said third and fourth region being capable of forming a double stranded RNA region; and
b) monitoring the reduction of the expression of said target gene by analyzing the reduction in expression of said second gene,
wherein said eukaryotic organism is a plant, a yeast, a fungus, a mold or an animal,
with the proviso that when said organism is an animal, the cell is not a cell in a human or animal body.

2. The method of claim 1, wherein said eukaryotic organism is selected from the group consisting of cotton, potato, corn, wheat, rice, sugar cane, oilseed rape, Arabidopsis, sugarbeet, tobacco and soybean.

3. The method of claim 1 or claim 2, wherein said eukaryotic organism is selected from the group consisting of insects, shellfish, molluscs, crustaceans, crabs, lobsters, prawns, fish, avian animals, mammals and humans.

4. The method of any one of claims 1 to 3, wherein said second gene is an endogenous gene present in said cell of said eukaryotic organism.

5. The method of any one of claims 1 to 3, wherein said second gene is a transgene stably integrated into the genome of said cell of said eukaryotic organism.

6. The method of claim 1 or claim 2, wherein said organism is a plant and said second gene is selected from the group consisting of PDS, EIN2, FLC and PhyB.

7. The method of any one of claims 1 to 3, wherein said cell of said eukaryotic organism comprises a functionally expressed GUS or a GFP gene, and said second gene is a GUS or GFP gene.

8. The method of any one of claims 1 to 7, wherein the double stranded RNA regions formed between the first and second region and the double stranded RNA region formed between the third and fourth region are about equal in size.

9. The method of any one of claims 1 to 7, wherein said first and second region, and said third and fourth region are about 300 nt in length.

10. The method of any one of claims 1 to 9, wherein said dsRNA is transcribed from a chimeric gene comprised within cells of said eukaryotic organism, said chimeric gene comprising:
a) a promoter region which functions in said eukaryotic cell; operably linked to
b) a DNA region which when transcribed yields said dsRNA molecule; and
c) a transcription termination and polyadenylation region that functions in said eukaryotic organism.

11. The method according to claim 10, wherein said chimeric gene is stably integrated into the genome of cells of said eukaryotic organism.

12. An RNA molecule as described in any one of claims 1 to 11, wherein the double stranded RNA regions formed between the first and second region and the double stranded RNA region formed between the third and fourth region are about equal in size.

13. Use of an RNA molecule according to claim 12 for measuring the reduction of expression of a target gene in a cell of a eukaryotic organism, wherein said eukaryotic organism is a plant, a yeast, a fungus, a mold or an animal, with the proviso that when said organism is an animal, the cell is not a cell in a human or animal body.

14. Use of an RNA molecule according to claim 12 to reduce the expression of said target gene and said second gene in a cell of a eukaryotic organism, wherein said eukaryotic organism is a plant, a yeast, a fungus, a mold or an animal, with the proviso that when said organism is an animal, the cell is not a cell in a human or animal body.

15. A DNA molecule for measuring the reduction of expression of a target gene in a cell of a eukaryotic organism comprising
a) a promoter region which functions in said eukaryotic cell; operably linked to
b) a DNA region which when transcribed yields a dsRNA molecule, said dsRNA comprising a first, second, third and fourth region;
i) said first region comprising a nucleotide sequence of at least 19 consecutive nucleotides having at least 94% sequence identity to 19 consecutive nucleotides from a sense nucleotide region of said target gene;
ii) said second region comprising a nucleotide sequence of at least 19 consecutive nucleotides having at least 94% sequence identity to a nucleotide sequence complementary to 19 consecutive nucleotides from said sense nucleotide region of the target gene ;
iii) said first region and said second region being capable of forming a double stranded RNA region:
iv) said third region comprising a nucleotide sequence of at least 19 consecutive nucleotides having at least 94% sequence identity to 19 consecutive nucleotides from a sense nucleotide region of a second gene present in said eukaryotic cell and which is different from said target gene;
v) said fourth region comprising a nucleotide sequence of at least 19 consecutive nucleotides having at least 94% sequence identity to the complement of said 19 consecutive nucleotides from said sense nucleotide region of said second gene;
vi) said third and fourth region being capable of forming a double stranded RNA region;
wherein the double stranded RNA regions formed between the first and second region and the double stranded RNA region formed between the third and fourth region are about equal in size; and
c) a transcription termination and polyadenylation region that functions in said eukaryotic organism, wherein said second gene is an endogenous gene of said eukaryotic organism or a transgene stably integrated into the genome of cells of said eukaryotic organism.

16. Use of a DNA molecule according to claim 15 to measure the expression of said target gene by measuring the reduction in expression of said second gene in a cell of a eukaryotic organism, wherein said eukaryotic organism is a plant, a yeast, a fungus, a mold or an animal, with the proviso that when said organism is an animal, the cell is not a cell in a human or animal body.

17. Use of a DNA molecule according to claim 15 to reduce the expression of said target gene and said second gene, in a cell of a eukaryotic organism, wherein said eukaryotic organism is a plant, a yeast, a fungus, a mold or an animal, with the proviso that when said organism is an animal, the cell is not a cell in a human or animal body.

18. A non-human eukaryotic organism comprising an RNA molecule according to claim 12.

19. A non-human eukaryotic organism comprising a DNA molecule according to claim 15.

20. The non-human eukaryotic organism according to claim 18 or 19, which is selected from a plant, an animal, a yeast, a fungus or a mold.

21. A method for identifying, within a population of dsRNA-mediated gene-silenced eukaryotic organisms, the organisms with the desired degree of silencing of a target gene comprising:
a) providing cells of said eukaryotic organisms with a dsRNA comprising a first region, a second region, a third region and a fourth region
i) said first region comprising a nucleotide sequence of at least 19 consecutive nucleotides having at least 94% sequence identity to 19 consecutive nucleotides from a sense nucleotide region of said target gene;
ii) said second region comprising a nucleotide sequence of at least 19 consecutive nucleotides having at least 94% sequence identity to a nucleotide sequence complementary to 19 consecutive nucleotides from said sense nucleotide region of the target gene ;
iii) said first region and said second region being capable of forming a double stranded RNA region:
iv) said third region comprising a nucleotide sequence of at least 19 consecutive nucleotides having at least 94% sequence identity to 19 consecutive nucleotides from a sense nucleotide region of a second gene present in said eukaryotic cell and which is different from said target gene;
v) said fourth region comprising a nucleotide sequence of at least 19 consecutive nucleotides having at least 94% sequence identity to the complement of said 19 consecutive nucleotides from said sense nucleotide region of said second gene;
vi) said third and fourth region being capable of forming a double stranded RNA region; and
b) identifying said organism with said desired degree of silencing of said target gene, by selecting said organisms with the desired degree of silencing of said second gene,
wherein said eukaryotic organism is a plant, a yeast, a fungus, a mold or an animal, with the proviso that when said organism is an animal, the cell is not a cell in a human or animal body.

22. The method according to claim 21, wherein the double stranded RNA regions formed between the first and second region and the double stranded RNA region formed between the third and fourth region are about equal in size.

## Patentansprüche

1. Verfahren zur Überwachung der Reduktion der Expression eines Target Gens in einer Zelle eines eukaryotischen Organismus, das genannte Verfahren umfasst die Schritte
a) zur Verfügung stellen der Zelle des genannten Organismus mit einer dsRNA, die eine erste Region, eine zweite Region, eine dritte Region und eine vierte Region umfasst,
i) die genannte erste Region umfasst eine Nukleotidsequenz von mindesten 19 aufeinanderfolgenden Nukleotiden, die mindestens eine 94%-ige Sequenzidentität zu 19 aufeinanderfolgenden Nukleotiden einer Sense-Nukleotidregion des genannten Target Gens aufweist;
ii) die genannte zweite Region umfasst eine Nukleotidsequenz von mindestens 19 aufeinanderfolgenden Nukleotiden, die eine mindestens 94%-ige Sequenzidentität zu einer Nukleotidsequenz aufweist, die komplementär zu 19 aufeinanderfolgenden Nukleotiden der genannten Sense-Nukleotidregion des genannten Target Gens ist;
iii) die genannte erste Region und die genannte zweite Region sind in der Lage eine doppelsträngige RNA-Region zu bilden;
iv) die genannte dritte Region umfasst eine Nukleotidsequenz von mindestens 19 aufeinanderfolgenden Nukleotiden, die eine mindestens 94%-ige Sequenzidentität zu 19 aufeinanderfolgenden Nukleotiden einer Sense-Nukleotidregion eines zweiten Gens, das in der genannten eukaryotischen Zelle vorhanden ist und das sich von dem genannten Target Gen unterscheidet, aufweist;
v) die genannte vierte Region umfasst eine Nukleotidsequenz von mindestens 19 aufeinanderfolgenden Nukleotiden, die eine mindestens 94%-ige Sequenzidentität zu den Komplementären der genannten aufeinanderfolgenden Nukleotiden der genannten Sense-Nukleotidregion von dem genannten zweiten Gen aufweist;
vi) die genannte dritte und vierte Region ist in der Lage eine doppelsträngige RNA-Region zu bilden; und
b) Überwachen der Reduktion der Expression des genannten Target Gens durch Analysieren der Reduktion der Expression des genannten zweiten Gens,
wobei der genannte eukaryotische Organismus eine Pflanze, eine Hefe, ein Pilz, Schimmel oder ein Tier ist, mit dem Vorbehalt, dass die Zelle keine Zelle in einem menschlichen oder tierischen Körper ist wenn der genannte Organismus ein Tier ist.

2. Verfahren nach Anspruch 1, wobei der genannte eukaryotische Organismus aus der Gruppe bestehend aus Baumwolle, Kartoffel, Mais, Weizen, Reis, Zuckerrohr, Ölsamenraps, Arabidopsis, Zuckerrübe, Tabak und Sojabohne ausgewählt ist.

3. Verfahren nach Anspruch 1 oder 2, wobei der genannte eukaryotische Organismus aus der Gruppe bestehend aus Insekten, Schalentieren, Weichtieren, Krustentieren, Krabben, Hummer, Garnelen, Fischen, Flugtieren, Säugetieren und Menschen ausgewählt ist.

4. Verfahren nach irgendeinem der Ansprüche 1 bis 3, wobei das genannte zweite Gen ein endogenes Gen ist, das in der genannten Zelle des genannten eukaryotischen Organismus vorhanden ist.

5. Verfahren nach irgendeinem der Ansprüche 1 bis 3, wobei das genannte zweite Gen ein Transgen ist, das stabil in das Genom der genannten Zelle des genannte eukaryotischen Organismus integriert ist.

6. Verfahren nach Anspruch 1 oder Anspruch 2, wobei der genannte Organismus eine Pflanze ist und das genannte zweite Gen aus der Gruppe bestehend aus PDS, EIN2, FLC und PhyB ausgewählt ist.

7. Verfahren nach irgendeinem der Ansprüche 1 bis 3, wobei die genannte Zelle des genannten eukaryotischen Organismus ein funktionell exprimiertes GUS oder ein GFP Gen umfasst, und das genannte zweite Gen ein GUS oder GFP Gen ist.

8. Verfahren nach irgendeinem der Ansprüche 1 bis 7, wobei die doppelsträngigen RNA-Regionen, die zwischen der ersten und zweiten Region gebildet werden
und die doppelsträngige RNA-Region, die zwischen der dritten und vierten Region gebildet wird, in etwa gleich groß sind.

9. Verfahren nach irgendeinem der Ansprüche 1 bis 7, wobei die genannte erste und zweite Region und die genannte dritte und vierte Region in etwa 300 nt an Länge aufweisen.

10. Verfahren nach irgendeinem der Ansprüche 1 bis 9, wobei das genannte dsRNA aus einem chimären Gen transkribiert ist, das in Zellen des genannten eukaryotischen Organismus umfasst ist, das genannte chimäre Gen umfasst:
a) eine Promoterregion, die in der genannten eukaryotischen Zelle wirkt; wirksam verknüpft mit
b) einer DNA-Region, die, wenn sie transkribiert wird, das genannte dsRNA Molekül ergibt; und
c) eine Transkriptionsterminations- und Polyadenylationsregion, die in dem genannten eukaryotischen Organismus wirkt.

11. Verfahren nach Anspruch 10, wobei das genannte chimäre Gen stabil in das Genom der Zellen des genannten eukaryotischen Organismus integriert ist.

12. RNA Molekül beschrieben wie in irgendeinem der Ansprüche 1 bis 11, wobei die doppelsträngigen RNA-Regionen, die zwischen der ersten und zweiten Region gebildet werden, und die doppelsträngige RNA-Region, die zwischen der dritten und vierten Region gebildet wird, in etwa gleich groß sind.

13. Verwendung eines RNA Moleküls nach Anspruch 12 zur Messung der Reduktion der Expression von einem Target Gen in einer Zelle eines eukaryotischen Organismus, wobei der genannte eukaryotische Organismus eine Pflanze, eine Hefe, ein Pilz, Schimmel oder ein Tier ist, mit dem Vorbehalt, dass die Zelle keine Zelle in einem menschlichen oder tierischen Körper ist wenn der genannte Organismus ein Tier ist.

14. Verwendung eines RNA Moleküls nach Anspruch 12 zur Reduktion der Expression des genannten Target Gens und des genannten zweiten Gens in einer Zelle eines eukaryotischen Organismus, wobei der genannte eukaryotische Organismus eine Pflanze, eine Hefe, ein Pilz, Schimmel oder ein Tier ist, mit dem Vorbehalt, dass die Zelle keine Zelle in einem menschlichen oder tierischen Körper ist wenn der genannte Organismus ein Tier ist.

15. DNA Molekül zur Messung der Reduktion der Expression eines Target Gens in einer Zelle eines eukaryotischen Organismus umfassend
a) eine Promoterregion, die in der genannten eukaryotischen Zelle wirkt; wirksam verknüpft mit
b) einer DNA-Region, die, wenn sie transkribiert wird, das genannte dsRNA Molekül ergibt; das genannte dsRNA umfasst eine erste, zweite, dritte und vierte Region;
i) die genannte erste Region umfasst eine Nukleotidsequenz von mindesten 19 aufeinanderfolgenden Nukleotiden, die mindestens eine 94%-ige Sequenzidentität zu 19 aufeinanderfolgenden Nukleotiden einer Sense-Nukleotidregion des genannten Target Gens aufweist;
ii) die genannte zweite Region umfasst eine Nukleotidsequenz von mindestens 19 aufeinanderfolgenden Nukleotiden, die eine mindestens 94%-ige Sequenzidentität zu einer Nukleotidsequenz aufweist, die komplementär zu 19 aufeinanderfolgenden Nukleotiden der genannten Sense-Nukleotidregion des genannten Target Gens ist;
iii) die genannte erste Region und die genannte zweite Region sind in der Lage eine doppelsträngige RNA-Region zu bilden;
iv) die genannte dritte Region umfasst eine Nukleotidsequenz von mindestens 19 aufeinanderfolgenden Nukleotiden, die eine mindestens 94%-ige Sequenzidentität zu 19 aufeinanderfolgenden Nukleotiden einer Sense-Nukleotidregion eines zweiten Gens, das in der genannten eukaryotischen Zelle vorhanden ist und das sich von dem genannten Target Gen unterscheidet, aufweist;
v) die genannte vierte Region umfasst eine Nukleotidsequenz von mindestens 19 aufeinanderfolgenden Nukleotiden, die eine mindestens 94%-ige Sequenzidentität zu den Komplementären der genannten aufeinanderfolgenden Nukleotiden der genannten Sense-Nukleotidregion von dem genannten zweiten Gen aufweist;
vi) die genannte dritte und vierte Region ist in der Lage eine doppelsträngige RNA-Region zu bilden; und
wobei die doppelsträngigen RNA-Regionen, die zwischen der ersten und zweiten Region gebildet werden und die doppelsträngige RNA-Region, die zwischen der dritten und vierten Region gebildet wird, in etwa gleich groß sind; und
c) eine Transkriptionsterminations- und Polyadenylationsregion, die in dem genannten eukaryotischen Organismus wirkt, wobei das genannte zweite Gen ein endogenes Gen des genannten eukaryotischen Organismus oder ein Transgen, das stabil in das Genom der Zellen des genannten eukaryotischen Organismus integriert ist, ist.

16. Verwendung eines DNA Moleküls nach Anspruch 15 zur Messung der Expression des genannte Target Gens durch Messung der Reduktion der Expression des genannten zweiten Gens in einer Zelle eines eukaryotischen Organismus, wobei der genannte eukaryotische Organismus eine Pflanze, eine Hefe, ein Pilz, Schimmel oder ein Tier ist, mit dem Vorbehalt, dass die Zelle keine Zelle in einem menschlichen oder tierischen Körper ist wenn der genannte Organismus ein Tier ist.

17. Verwendung eines DNA Moleküls nach Anspruch 15, um die Expression des genannten Target Gens und des genannten zweiten Gens in einer Zelle des eukaryotischen Organismus zu reduzieren, wobei der genannte eukaryotische Organismus eine Pflanze, eine Hefe, ein Pilz, Schimmel oder ein Tier ist, mit dem Vorbehalt, dass die Zelle keine Zelle in einem menschlichen oder tierischen Körper ist wenn der genannte Organismus ein Tier ist.

18. Nicht-humaner eukaryotischer Organismus, der ein RNA Molekül nach Anspruch 12 umfasst.

19. Nicht-humaner eukaryotischer Organismus, der ein RNA Molekül nach Anspruch 15 umfasst.

20. Nicht-humaner eukaryotischer Organismus nach Anspruch 18 oder 19, der aus einer Pflanze, einem Tier, einer Hefe, einem Pilz oder Schimmel ausgewählt ist.

21. Verfahren zur Identifizierung des Organismus mit dem gewünschten Silencing-Grad eines Target Gens innerhalb einer Population von dsRNA-vermittelten Gen-silenced eukaryotischen Organismen, umfassend:
a) zur Verfügung stellen der Zelle des genannten Organismus mit einer dsRNA, die eine erste Region, eine zweite Region, eine dritte Region und eine vierte Region umfasst,
i) die genannte erste Region umfasst eine Nukleotidsequenz von mindesten 19 aufeinanderfolgenden Nukleotiden, die mindestens eine 94%-ige Sequenzidentität zu 19 aufeinanderfolgenden Nukleotiden einer Sense Nukleotidregion des genannten Target Gens aufweist;
ii) die genannte zweite Region umfasst eine Nukleotidsequenz von mindestens 19 aufeinanderfolgenden Nukleotiden, die eine mindestens 94%-ige Sequenzidentität zu einer Nukleotidsequenz aufweist, die komplementär zu 19 aufeinanderfolgenden Nukleotiden der genannten Sense-Nukleotidregion des genannten Target Gens ist;
iii) die genannte erste Region und die genannte zweite Region sind in der Lage eine doppelsträngige RNA-Region zu bilden;
iv) die genannte dritte Region umfasst eine Nukleotidsequenz von mindestens 19 aufeinanderfolgenden Nukleotiden, die eine mindestens 94%-ige Sequenzidentität zu 19 aufeinanderfolgenden Nukleotiden einer Sense-Nukleotidregion eines zweiten Gens, das in der genannten eukaryotischen Zelle vorhanden ist und das sich von dem genannten Target Gen unterscheidet, aufweist;
v) die genannte vierte Region umfasst eine Nukleotidsequenz von mindestens 19 aufeinanderfolgenden Nukleotiden, die eine mindestens 94%-ige Sequenzidentität zu den Komplementären der genannten aufeinanderfolgenden Nukleotiden der genannten Sense-Nukleotidregion von dem genannten zweiten Gen aufweist;
vi) die genannte dritte und vierte Region ist in der Lage eine doppelsträngige RNA-Region zu bilden; und
b) Identifizieren des genannten Organismus mit dem genannten gewünschten Silencing-Grad des genannten Target Gens, durch Selektieren des genannten Organismus mit dem gewünschten Silencing-Grad des genannten zweiten Gens,
wobei der genannte eukaryotische Organismus eine Pflanze, eine Hefe, ein Pilz, Schimmel oder ein Tier ist, mit dem Vorbehalt, dass die Zelle keine Zelle in einem menschlichen oder tierischen Körper ist wenn der genannte Organismus ein Tier ist.

22. Verfahren nach Anspruch 21, wobei die doppelsträngigen RNA-Regionen, die zwischen der ersten und zweiten Region gebildet werden und die doppelsträngige RNA-Region, die zwischen der dritten und vierten Region gebildet wird, in etwa gleich groß sind.

## Revendications

1. Procédé de suivi de la réduction de l'expression d'un gène cible dans une cellule d'un organisme eucaryote, ledit procédé comprenant les étapes de
a) introduction dans la cellule dudit organisme eucaryote d'un ARN à double brin comprena nt une première région, une deuxième région, une troisième région et une quatrième région
i) ladite première région comprenant une séquence nucléotidique d'au moins 19 nucléotides consécutifs ayant au moins 94% d'identité de séquence avec 19 nucléotides consécutifs d'une région de nucléotides sens dudit gène cible ;
ii) ladite deuxième région comprenant une séquence nucléotidique d'au moins 19 nucléotides consécutifs ayant au moins 94% d'identité de séquence avec une séquence nucléotidique complémentaire à 19 nucléotides consécutifs de ladite région de nucléotides sens du gène cible ;
iii) ladite première région et ladite deuxième région étant aptes à former une région d'ARN à double brin ;
iv) ladite troisième région comprenant une séquence nucléotidique d'au moins 19 nucléotides consécutifs ayant au moins 94% d'identité de séquence avec 19 nucléotides consécutifs d'une région de nucléotides sens d'un deuxième gène présent dans ladite cellule eucaryote et qui est différent dudit gène cible ;
v) ladite q uatrième région comprenant une séquence nucléotidique d'au moins 19 nucléotides consécutifs ayant au moins 94% d'identité de séquence avec le complément desdits 19 nucléotides consécutifs de ladite région de nucléotides sens dudit deuxième gène ;
vi) lesdites troisième et quatrième régions étant aptes à former une région d'ARN à double brin ; et
b) suivi de la réduction de l'expression dudit gène cible en analysant la réduction de l'expression dudit deuxième gène,
dans lequel ledit organisme eucaryote es t une plante, une levure, un champignon, une moisissure ou un animal, sous réserve que lorsque ledit organisme est un animal, la cellule n'est pas une cellule dans un corps humain ou animal.

2. Procédé selon la revendication 1, dans lequel ledit organisme eucaryote est choisi parmi le groupe consistant en le coton, la pomme de terre, le maïs, le blé, le riz, la canne à sucre, le colza, *Arabidopsis,* la betterave à sucre, le tabac et le soja.

3. Procédé selon la revendication 1 ou la revendication 2, dans 1 equel ledit organisme eucaryote est choisi parmi le groupe consistant en des insectes, des fruits de mer, des mollusques, des crustacés, des crabes, des homards, des crevettes , des poissons, des animaux aviaires, des mammifères et des humains.

4. Procédé selon l'une quelconque des revendications 1 à 3, dans lequel ledit deuxième gène est un gène endogène présent dans ladite cellule dudit organisme eucaryote.

5. Procédé selon l'une quelconque des revendications 1 à 3, dans lequel ledit deuxième gène est un transgène intégré de façon stable dans le génome de ladite cellule dudit organisme eucaryote.

6. Procédé selon la revendication 1 ou la revendication 2, dans lequel ledit organisme est une plante et ledit deuxième gène est choisi parmi le groupe consistant en PDS, EIN2, FLC et PhyB.

7. Procédé selon l'une quelconque des revendications 1 à 3, dans lequel ladite cellule dudit organisme eucaryote comprend un gène GUS ou GFP exprimé fonctionnellement, et ledit deuxième gène est un gène GUS ou GFP.

8. Procédé selon l'une quelconque des revendications 1 à 7, dans lequel les régions d'ARN à double brin formées entre la première et la deuxième région et la région d'ARN à double brin formée entre la troisième et la quatrième région sont approximativement égales en taille.

9. Procédé selon l'une quelconque des revendications 1 à 7, dans lequel lesdites première et deuxième région, et lesdites troisième et quatrième région sont d'environ 300 nt en longueur.

10. Procédé selon l'une quelconque des revendications 1 à 9, dans lequel ledit ARN à double brin est transcrit à partir d'un gène chimère compris à l'intérieur de cellules dudit organisme eucaryote, ledit gène chimère comprenant :
a) une région de promoteur qui fonctionne dans ladite cellule eucaryote ; opérationnellement liée à
b) une région d'ADN qui, lorsqu'elle est transcrite, donne ladite molécule d'ARN à double brin ; et
c) une région de terminaison de transcription et de polyadénylation qui fonctionne dans ledit organisme eucaryote.

11. Procédé selon 1 a revendication 10, dans lequel ledit gène chimère est intégré de façon stable dans le génome de cellules dudit organisme eucaryote.

12. Molécule d'ARN telle que décrite dans l'une quelconque des revendications 1 à 11, dans laquelle les régions d'ARN à double brin formées entre la première et la deuxième région et la région d'ARN à double brin formée entre la troisième et la quatrième région sont approximativement égales en taille.

13. Utilisation d'une molécule d'ARN selon la revendication 12 pour mesure r la réduction de l'expression d'un gène cible dans une cellule d'un organisme eucaryote dans laquelle ledit organisme eucaryote est une plante, une levure, un champignon, une moisissure ou un animal, sous réserve que lorsque ledit organisme est un animal , la cellule n'est pas une cellule dans un corps humain ou animal.

14. Utilisation d'une molécule d'ARN selon la revendication 12 pour réduire l'expression dudit gène cible et dudit deuxième gène dans une cellule d'un organisme eucaryote, dans laquelle ledit organisme eucaryote est une plante, une levure, un champignon, une moisissure ou un animal, sous réserve que lorsque ledit organisme est un animal, la cellule n'est pas une cellule dans un corps humain ou animal.

15. Molécule d'ADN pour mesurer la réd uction de l'expression d'un gène cible dans une cellule d'un organisme eucaryote comprenant
a) une région de promoteur qui fonctionne dans ladite cellule eucaryote ; opérationnellement liée à
b) une région d'ADN qui, lorsqu'elle est transcrite, donne une molécule d'ARN à double brin , ladite molécule d'ARN à double brin comprenant une première, une deuxième, une troisième et une quatrième région ;
i) ladite première région comprenant une séquence nucléotidique d'au moins 19 nucléotides consécutifs ayant au moins 94% d'identité de séquence avec 19 nucléotides consécutifs d'une région de nucléotides sens dudit gène cible ;
ii) ladite deuxième région comprenant une séquence nucléotidique d'au moins 19 nucléotides consécutifs ayant au moins 94% d'identité de séquence avec une séquence nucléotidique complémentaire à 19 nucléotides consécutifs de ladite région de nucléotides sens du gène cible ;
iii) ladite première région et ladite deuxième région étant aptes à former une région d'ARN à double brin ;
iv) ladite troisième région comprenant une séquence nucléotidique d'au moins 19 nucléotides consécutifs ayant au moins 94% d'identité de séquence avec 19 nucléotides consécutifs d'une région de nucléotides sens d'un deuxième gène présent dans ladite cellule eucary ote et qui est différent dudit gène cible ;
v) ladite quatrième région comprenant une séquence nucléotidique d'au moins 19 nucléotides consécutifs ayant au moins 94% d'identité de séquence avec le complément desdits 19 nucléotides consécutifs de ladite région de nucléotides sens dudit deuxième gène ;
vi) lesdites troisième et quatrième régions étant aptes à former une région d'ARN à double brin ;
dans laquelle les régions d'ARN à double brin formées entre la première et la deuxième région et la région d'ARN à double brin formée entre la troisième et la quatrième région sont approximativement égales en taille ; et
c) une région de terminaison de transcription et de polyadénylation qui fonctionne dans ledit organisme eucaryote, dans laquelle ledit deuxième gène est un gène endogène dudit organisme eucaryote ou un transgène intégré de façon stable dans le génome de cellules dudit organisme eucaryote.

16. Utilisation d'une molécule d'ADN selon la revendication 15 pour mesurer l'expression dudit gène cible en mesurant la réduction de l'expression dudit deuxième gène dans une cellule d'un organisme eucaryote, dans laquelle ledit organisme eucaryote est une plante, une levure, un champignon, une moisissure ou un animal, sous réserve que lorsque ledit organisme e st un animal, la cellule n'est pas une cellule dans un corps humain ou animal.

17. Utilisation d'une molécule d'ADN selon la revendication 15 pour réduire l'expression dudit gène cible et dudit deuxième gène, dans une cellule d'un organisme eucaryote, dans laquelle ledit organisme eucaryote est une plante, une levure, un champignon, une moisissure ou un animal, sous réserve que lorsque ledit organisme est un animal, la cellule n'est pas une cellule dans un corps humain ou animal.

18. Organisme eucaryote n on humain comprenant une molécule d'ARN selon la revendication 12.

19. Organisme eucaryote non humain comprenant une molécule d'ADN selon la revendication 15.

20. Organisme eucaryote non humain selon la revendication 18 ou 19, qui est choisi parmi une plante, un animal, une levure, un champignon ou une moisissure.

21. Procédé d'identification, au sein d'une population d'organismes eucaryotes à gènes rendus silencieux par l'intermédiaire d' ARN à double brin, des organismes présentant le degré désiré de si lençage d'un gène cible, comprenant :
a) l'introduction dans des cellule s des dits organismes eucaryotes d'un ARN à double brin comprenant une première région, une deuxième région, une troisième région et une quatrième région
i) ladite première région comprenant une séquence nucléotidique d'au moins 19 nucléotides consécutifs ayant au moins 94% d'identité de séquence avec 19 nucléotides consécutifs d'une région de nucléotides sens dudit gène cible ;
ii) ladite deuxième région comprenant une séquence nuclé otidique d'au moins 19 nucléotides consécutifs ayant au moins 94% d'identité de séquence avec une séquence nucléotidique complémentaire à 19 nucléotides consécutifs de ladite région de nucléotides sens du gène cible ;
iii) ladite première région et ladite deuxième région étant aptes à former une région d'ARN à double brin ;
iv) ladite troisième région comprenant une séquence nucléotidique d'au moins 19 nucléotides consécutifs ayant au moins 94% d'identité de séquence avec 19 nucléotides consécutifs d'une région de nucléotides sens d'un deuxième gène présent dans ladite cellule eucaryote et qui est différent dudit gène cible ;
v) ladite quatrième région comprenant une séquence nucléotidique d'au moins 19 nucléotides consécutifs ayant au moins 94% d'identit é de séquence avec le complément desdits 19 nucléotides consécutifs de ladite région de nucléotides sens dudit deuxième gène ;
vi) lesdites troisième et quatrième régions étant aptes à former une région d'ARN à double brin ; et
b) l'identification dudit organisme présentant le degré désiré de silençage dudit gène cible, en sélectionnant lesdits organismes présentant le degré désiré de silençage dudit deuxième gène,
dans lequel ledit organisme eucaryote est une plante, une levure, un champignon, une moisi ssure ou un animal, sous réserve que lorsque ledit organisme est un animal, la cellule n'est pas une cellule dans un corps humain ou animal.

22. Procédé selon la revendication 21, dans lequel les régions d'ARN à double brin formées entre la première et la deuxième région et la région d'ARN à double brin formée entre la troisième et la quatrième région sont approximativement égales en taille.


### SEQUENCE LISTING

<110> Commonwealth Scientific and Industrial Research Organization
<120> Methods and means for monitoring and modulating gene silencing
<130> COLINA-PCT
<150> US 60363852
<151> 2003-03-14
<160> 7
<170> PatentIn version 3.0
<210> 1
   <211> 33
   <212> DNA
   <213> Artificial
<220>
   <223> olignucleotide primer for PCR amplification of CHS/FLC fragment
<400> 1
   ctcgagtcta gaggagcaga agctgagatg gag 33
<210> 2
   <211> 27
   <212> DNA
   <213> Artificial
<220>
   <223> olignucleotide primer for PCR amplification of CHS/FLC fragment
<400> 2
   ctgcaggaat aaggtacaaa gttcatc 27
<210> 3
   <211> 27
   <212> DNA
   <213> Artificial
<220>
   <223> olignucleotide primer for PCR amplification of CHS/FLC fragment
<400> 3
   ctgcaggcac tgctaaccct gagaaac 27
<210> 4
   <211> 27
   <212> DNA
   <213> Artificial
<220>
   <223> olignucleotide primer for PCR amplification of CHS/FLC fragment
<400> 4
   ggtaccttga cttgggctgg ccccact 27
<210> 5
   <211> 29
   <212> DNA
   <213> Artificial
<220>
   <223> attB1 recombination site
<400> 5
   ggggacaagt ttgtacaaaa aagcaggct 29
<210> 6
   <211> 27
   <212> DNA
   <213> Artificial
<220>
   <223> attB2 recombination site
<400> 6
   ggaccacttt gtacaagaaa gctgggt 27
<210> 7
   <211> 17476
   <212> DNA
   <213> Artificial
<220>
   <223> plasmid pHELLSGATE 8
<400> 7
   ggccgcacta gtgatatccc gcggccatgg cggccgggag catgcgacgt cgggcccaat    60
   tcgccctata gtgagtcgta ttacaattca ctggccgtcg ttttacaacg tcgtgactgg   120
   gaaaaccctg gcgttaccca acttaatcgc cttgcagcac atcccccttt cgccagctgg   180
   cgtaatagcg aagaggcccg caccgatcgc ccttcccaac agttgcgcag cctgaatggc   240
   gaatggaaat tgtaaacgtt aatgggtttc tggagtttaa tgagctaagc acatacgtca   300
   gaaaccatta ttgcgcgttc aaaagtcgcc taaggtcact atcagctagc aaatatttct   360
   tgtcaaaaat gctccactga cgttccataa attcccctcg gtatccaatt agagtctcat   420
   attcactctc aatccaaata atctgcaatg gcaattacct tatccgcaac ttctttacct   480
   atttccgccc ggatccgggc aggttctccg gccgcttggg tggagaggct attcggctat   540
   gactgggcac aacagacaat cggctgctct gatgccgccg tgttccggct gtcagcgcag   600
   gggcgcccgg ttctttttgt caagaccgac ctgtccggtg ccctgaatga actgcaggac   660
   gaggcagcgc ggctatcgtg gctggccacg acgggcgttc cttgcgcagc tgtgctcgac   720
   gttgtcactg aagcgggaag ggactggctg ctattgggcg aagtgccggg gcaggatctc   780
   ctgtcatctc accttgctcc tgccgagaaa gtatccatca tggctgatgc aatgcggcgg   840
   ctgcatacgc ttgatccggc tacctgccca ttcgaccacc aagcgaaaca tcgcatcgag   900
   cgagcacgta ctcggatgga agccggtctt gtcgatcagg atgatctgga cgaagagcat   960
   caggggctcg cgccagccga actgttcgcc aggctcaagg cgcgcatgcc cgacggcgag  1020
   gatctcgtcg tgacccatgg cgatgcctgc ttgccgaata tcatggtgga aaatggccgc  1080
   ttttctggat tcatcgactg tggccggctg ggtgtggcgg accgctatca ggacatagcg  1140
   ttggctaccc gtgatattgc tgaagagctt ggcggcgaat gggctgaccg cttcctcgtg  1200
   ctttacggta tcgccgctcc cgattcgcag cgcatcgcct tctatcgcct tcttgacgag  1260
   ttcttctgag cgggactctg gggttcgaaa tgaccgacca agcgacgccc aacctgccat  1320
   cacgagattt cgattccacc gccgccttct atgaaaggtt gggcttcgga atcgttttcc  1380
   gggacgccgg ctggatgatc ctccagcgcg gggatctcat gctggagttc ttcgcccacc  1440
   ccgatccaac acttacgttt gcaacgtcca agagcaaata gaccacgaac gccggaaggt  1500
   tgccgcagcg tgtggattgc gtctcaattc tctcttgcag gaatgcaatg atgaatatga  1560
   tactgactat gaaactttga gggaatactg cctagcaccg tcacctcata acgtgcatca  1620
   tgcatgccct gacaacatgg aacatcgcta tttttctgaa gaattatgct cgttggagga  1680
   tgtcgcggca attgcagcta ttgccaacat cgaactaccc ctcacgcatg cattcatcaa  1740
   tattattcat gcggggaaag gcaagattaa tccaactggc aaatcatcca gcgtgattgg  1800
   taacttcagt tccagcgact tgattcgttt tggtgctacc cacgttttca ataaggacga  1860
   gatggtggag taaagaagga gtgcgtcgaa gcagatcgtt caaacatttg gcaataaagt  1920
   ttcttaagat tgaatcctgt tgccggtctt gcgatgatta tcatataatt tctgttgaat  1980
   tacgttaagc atgtaataat taacatgtaa tgcatgacgt tatttatgag atgggttttt  2040
   atgattagag tcccgcaatt atacatttaa tacgcgatag aaaacaaaat atagcgcgca  2100
   aactaggata aattatcgcg cgcggtgtca tctatgttac tagatcgaat taattccagg  2160
   cggtgaaggg caatcagctg ttgcccgtct cactggtgaa aagaaaaacc accccagtac 2220
   attaaaaacg tccgcaatgt gttattaagt tgtctaagcg tcaatttgtt tacaccacaa 2280
   tatatcctgc caccagccag ccaacagctc cccgaccggc agctcggcac aaaatcacca 2340
   ctcgatacag gcagcccatc agtccgggac ggcgtcagcg ggagagccgt tgtaaggcgg 2400
   cagactttgc tcatgttacc gatgctattc ggaagaacgg caactaagct gccgggtttg 2460
   aaacacggat gatctcgcgg agggtagcat gttgattgta acgatgacag agcgttgctg 2520
   cctgtgatca aatatcatct ccctcgcaga gatccgaatt atcagccttc ttattcattt 2580
   ctcgcttaac cgtgacaggc tgtcgatctt gagaactatg ccgacataat aggaaatcgc 2640
   tggataaagc cgctgaggaa gctgagtggc gctatttctt tagaagtgaa cgttgacgat 2700
   gtcgacggat cttttccgct gcataaccct gcttcggggt cattatagcg attttttcgg 2760
   tatatccatc ctttttcgca cgatatacag gattttgcca aagggttcgt gtagactttc 2820
   cttggtgtat ccaacggcgt cagccgggca ggataggtga agtaggccca cccgcgagcg 2880
   ggtgttcctt cttcactgtc ccttattcgc acctggcggt gctcaacggg aatcctgctc 2940
   tgcgaggctg gccggctacc gccggcgtaa cagatgaggg caagcggatg gctgatgaaa 3000
   ccaagccaac caggggtgat gctgccaact tactgattta gtgtatgatg gtgtttttga 3060
   ggtgctccag tggcttctgt ttctatcagc tgtccctcct gttcagctac tgacggggtg 3120
   gtgcgtaacg gcaaaagcac cgccggacat cagcgctatc tctgctctca ctgccgtaaa 3180
   acatggcaac tgcagttcac ttacaccgct tctcaacccg gtacgcacca gaaaatcatt 3240
   gatatggcca tgaatggcgt tggatgccgg gcaacagccc gcattatggg cgttggcctc 3300
   aacacgattt tacgtcactt aaaaaactca ggccgcagtc ggtaacctcg cgcatacagc 3360
   cgggcagtga cgtcatcgtc tgcgcggaaa tggacgaaca gtggggctat gtcggggcta 3420
   aatcgcgcca gcgctggctg ttttacgcgt atgacagtct ccggaagacg gttgttgcgc 3480
   acgtattcgg tgaacgcact atggcgacgc tggggcgtct tatgagcctg ctgtcaccct 3540
   ttgacgtggt gatatggatg acggatggct ggccgctgta tgaatcccgc ctgaagggaa 3600
   agctgcacgt aatcagcaag cgatatacgc agcgaattga gcggcataac ctgaatctga 3660
   ggcagcacct ggcacggctg ggacggaagt cgctgtcgtt ctcaaaatcg gtggagctgc 3720
   atgacaaagt catcgggcat tatctgaaca taaaacacta tcaataagtt ggagtcatta 3780
   cccaaccagg aagggcagcc cacctatcaa ggtgtactgc cttccagacg aacgaagagc 3840
   gattgaggaa aaggcggcgg cggccggcat gagcctgtcg gcctacctgc tggccgtcgg 3900
   ccagggctac aaaatcacgg gcgtcgtgga ctatgagcac gtccgcgagc tggcccgcat 3960
   caatggcgac ctgggccgcc tgggcggcct gctgaaactc tggctcaccg acgacccgcg 4020
   cacggcgcgg ttcggtgatg ccacgatcct cgccctgctg gcgaagatcg aagagaagca 4080
   ggacgagctt ggcaaggtca tgatgggcgt ggtccgcccg agggcagagc catgactttt 4140
   ttagccgcta aaacggccgg ggggtgcgcg tgattgccaa gcacgtcccc atgcgctcca 4200
   tcaagaagag cgacttcgcg gagctggtat tcgtgcaggg caagattcgg aataccaagt 4260
   acgagaagga cggccagacg gtctacggga ccgacttcat tgccgataag gtggattatc 4320
   tggacaccaa ggcaccaggc gggtcaaatc aggaataagg gcacattgcc ccggcgtgag 4380
   tcggggcaat cccgcaagga gggtgaatga atcggacgtt tgaccggaag gcatacaggc 4440
   aagaactgat cgacgcgggg ttttccgccg aggatgccga aaccatcgca agccgcaccg 4500
   tcatgcgtgc gccccgcgaa accttccagt ccgtcggctc gatggtccag caagctacgg 4560
   ccaagatcga gcgcgacagc gtgcaactgg ctccccctgc cctgcccgcg ccatcggccg 4620
   ccgtggagcg ttcgcgtcgt ctcgaacagg aggcggcagg tttggcgaag tcgatgacca 4680
   tcgacacgcg aggaactatg acgaccaaga agcgaaaaac cgccggcgag gacctggcaa 4740
   aacaggtcag cgaggccaag caggccgcgt tgctgaaaca cacgaagcag cagatcaagg 4800
   aaatgcagct ttccttgttc gatattgcgc cgtggccgga cacgatgcga gcgatgccaa 4860
   acgacacggc ccgctctgcc ctgttcacca cgcgcaacaa gaaaatcccg cgcgaggcgc 4920
   tgcaaaacaa ggtcattttc cacgtcaaca aggacgtgaa gatcacctac accggcgtcg 4980
   agctgcgggc cgacgatgac gaactggtgt ggcagcaggt gttggagtac gcgaagcgca 5040
   cccctatcgg cgagccgatc accttcacgt tctacgagct ttgccaggac ctgggctggt 5100
   cgatcaatgg ccggtattac acgaaggccg aggaatgcct gtcgcgccta caggcgacgg 5160
   cgatgggctt cacgtccgac cgcgttgggc acctggaatc ggtgtcgctg ctgcaccgct 5220
   tccgcgtcct ggaccgtggc aagaaaacgt cccgttgcca ggtcctgatc gacgaggaaa 5280
   tcgtcgtgct gtttgctggc gaccactaca cgaaattcat atgggagaag taccgcaagc 5340
   tgtcgccgac ggcccgacgg atgttcgact atttcagctc gcaccgggag ccgtacccgc 5400
   tcaagctgga aaccttccgc ctcatgtgcg gatcggattc cacccgcgtg aagaagtggc 5460
   gcgagcaggt cggcgaagcc tgcgaagagt tgcgaggcag cggcctggtg gaacacgcct 5520
   gggtcaatga tgacctggtg cattgcaaac gctagggcct tgtggggtca gttccggctg 5580
   ggggttcagc agccagcgct ttactggcat ttcaggaaca agcgggcact gctcgacgca 5640
   cttgcttcgc tcagtatcgc tcgggacgca cggcgcgctc tacgaactgc cgataaacag 5700
   aggattaaaa ttgacaattg tgattaaggc tcagattcga cggcttggag cggccgacgt 5760
   gcaggatttc cgcgagatcc gattgtcggc cctgaagaaa gctccagaga tgttcgggtc 5820
   cgtttacgag cacgaggaga aaaagcccat ggaggcgttc gctgaacggt tgcgagatgc 5880
   cgtggcattc ggcgcctaca tcgacggcga gatcattggg ctgtcggtct tcaaacagga 5940
   ggacggcccc aaggacgctc acaaggcgca tctgtccggc gttttcgtgg agcccgaaca 6000
   gcgaggccga ggggtcgccg gtatgctgct gcgggcgttg ccggcgggtt tattgctcgt 6060
   gatgatcgtc cgacagattc caacgggaat ctggtggatg cgcatcttca tcctcggcgc 6120
   acttaatatt tcgctattct ggagcttgtt gtttatttcg gtctaccgcc tgccgggcgg 6180
   ggtcgcggcg acggtaggcg ctgtgcagcc gctgatggtc gtgttcatct ctgccgctct 6240
   gctaggtagc ccgatacgat tgatggcggt cctgggggct atttgcggaa ctgcgggcgt 6300
   ggcgctgttg gtgttgacac caaacgcagc gctagatcct gtcggcgtcg cagcgggcct 6360
   ggcgggggcg gtttccatgg cgttcggaac cgtgctgacc cgcaagtggc aacctcccgt 6420
   gcctctgctc acctttaccg cctggcaact ggcggccgga ggacttctgc tcgttccagt 6480
   agctttagtg tttgatccgc caatcccgat gcctacagga accaatgttc tcggcctggc 6540
   gtggctcggc ctgatcggag cgggtttaac ctacttcctt tggttccggg ggatctcgcg 6600
   actcgaacct acagttgttt ccttactggg ctttctcagc cgggatggcg ctaagaagct 6660
   attgccgccg atcttcatat gcggtgtgaa ataccgcaca gatgcgtaag gagaaaatac 6720
   cgcatcaggc gctcttccgc ttcctcgctc actgactcgc tgcgctcggt cgttcggctg 6780
   cggcgagcgg tatcagctca ctcaaaggcg gtaatacggt tatccacaga atcaggggat 6840
   aacgcaggaa agaacatgtg agcaaaaggc cagcaaaagg ccaggaaccg taaaaaggcc 6900
   gcgttgctgg cgtttttcca taggctccgc ccccctgacg agcatcacaa aaatcgacgc 6960
   tcaagtcaga ggtggcgaaa cccgacagga ctataaagat accaggcgtt tccccctgga 7020
   agctccctcg tgcgctctcc tgttccgacc ctgccgctta ccggatacct gtccgccttt 7080
   ctcccttcgg gaagcgtggc gctttctcaa tgctcacgct gtaggtatct cagttcggtg 7140
   taggtcgttc gctccaagct gggctgtgtg cacgaacccc ccgttcagcc cgaccgctgc 7200
   gccttatccg gtaactatcg tcttgagtcc aacccggtaa gacacgactt atcgccactg 7260
   gcagcagcca ctggtaacag gattagcaga gcgaggtatg taggcggtgc tacagagttc 7320
   ttgaagtggt ggcctaacta cggctacact agaaggacag tatttggtat ctgcgctctg 7380
   ctgaagccag ttaccttcgg aaaaagagtt ggtagctctt gatccggcaa acaaaccacc 7440
   gctggtagcg gtggtttttt tgtttgcaag cagcagatta cgcgcagaaa aaaaggatat 7500
   caagaagatc ctttgatctt ttctacgggg tctgacgctc agtggaacga aaactcacgt 7560
   taagggattt tggtcatgag attatcaaaa aggatcttca cctagatcct tttaaattaa 7620
   aaatgaagtt ttaaatcaat ctaaagtata tatgagtaaa cttggtctga cagttaccaa 7680
   tgcttaatca gtgaggcacc tatctcagcg atctgtctat ttcgttcatc catagttgcc 7740
   tgactccccg tcgtgtagat aactacgata cgggagggct taccatctgg ccccagtgct 7800
   gcaatgatac cgcgagaccc acgctcaccg gctccagatt tatcagcaat aaaccagcca 7860
   gccggaaggg ccgagcgcag aagtggtcct gcaactttat ccgcctccat ccagtctatt 7920
   aaacaagtgg cagcaacgga ttcgcaaacc tgtcacgcct tttgtgccaa aagccgcgcc 7980
   aggtttgcga tccgctgtgc caggcgttag gcgtcatatg aagatttcgg tgatccctga 8040
   gcaggtggcg gaaacattgg atgctgagaa ccatttcatt gttcgtgaag tgttcgatgt 8100
   gcacctatcc gaccaaggct ttgaactatc taccagaagt gtgagcccct accggaagga 8160
   ttacatctcg gatgatgact ctgatgaaga ctctgcttgc tatggcgcat tcatcgacca 8220
   agagcttgtc gggaagattg aactcaactc aacatggaac gatctagcct ctatcgaaca 8280
   cattgttgtg tcgcacacgc accgaggcaa aggagtcgcg cacagtctca tcgaatttgc 8340
   gaaaaagtgg gcactaagca gacagctcct tggcatacga ttagagacac aaacgaacaa 8400
   tgtacctgcc tgcaatttgt acgcaaaatg tggctttact ctcggcggca ttgacctgtt 8460
   cacgtataaa actagacctc aagtctcgaa cgaaacagcg atgtactggt actggttctc 8520
   gggagcacag gatgacgcct aacaattcat tcaagccgac accgcttcgc ggcgcggctt 8580
   aattcaggag ttaaacatca tgagggaagc ggtgatcgcc gaagtatcga ctcaactatc 8640
   agaggtagtt ggcgtcatcg agcgccatct cgaaccgacg ttgctggccg tacatttgta 8700
   cggctccgca gtggatggcg gcctgaagcc acacagtgat attgatttgc tggttacggt 8760
   gaccgtaagg cttgatgaaa caacgcggcg agctttgatc aacgaccttt tggaaacttc 8820
   ggcttcccct ggagagagcg agattctccg cgctgtagaa gtcaccattg ttgtgcacga 8880
   cgacatcatt ccgtggcgtt atccagctaa gcgcgaactg caatttggag aatggcagcg 8940
   caatgacatt cttgcaggta tcttcgagcc agccacgatc gacattgatc tggctatctt 9000
   gctgacaaaa gcaagagaac atagcgttgc cttggtaggt ccagcggcgg aggaactctt 9060
   tgatccggtt cctgaacagg atctatttga ggcgctaaat gaaaccttaa cgctatggaa 9120
   ctcgccgccc gactgggctg gcgatgagcg aaatgtagtg cttacgttgt cccgcatttg 9180
   gtacagcgca gtaaccggca aaatcgcgcc gaaggatgtc gctgccgact gggcaatgga 9240
   gcgcctgccg gcccagtatc agcccgtcat acttgaagct aggcaggctt atcttggaca 9300
   agaagatcgc ttggcctcgc gcgcagatca gttggaagaa tttgttcact acgtgaaagg 9360
   cgagatcacc aaggtagtcg gcaaataatg tctaacaatt cgttcaagcc gacgccgctt 9420
   cgcggcgcgg cttaactcaa gcgttagaga gctggggaag actatgcgcg atctgttgaa 9480
   ggtggttcta agcctcgtac ttgcgatggc atcggggcag gcacttgctg acctgccaat 9540
   tgttttagtg gatgaagctc gtcttcccta tgactactcc ccatccaact acgacatttc 9600
   tccaagcaac tacgacaact ccataagcaa ttacgacaat agtccatcaa attacgacaa 9660
   ctctgagagc aactacgata atagttcatc caattacgac aatagtcgca acggaaatcg 9720
   taggcttata tatagcgcaa atgggtctcg cactttcgcc ggctactacg tcattgccaa 9780
   caatgggaca acgaacttct tttccacatc tggcaaaagg atgttctaca ccccaaaagg 9840
   ggggcgcggc gtctatggcg gcaaagatgg gagcttctgc ggggcattgg tcgtcataaa 9900
   tggccaattt tcgcttgccc tgacagataa cggcctgaag atcatgtatc taagcaacta   9960
   gcctgctctc taataaaatg ttaggagctt ggctgccatt tttggggtga ggccgttcgc  10020
   ggccgagggg cgcagcccct ggggggatgg gaggcccgcg ttagcgggcc gggagggttc  10080
   gagaaggggg ggcacccccc ttcggcgtgc gcggtcacgc gccagggcgc agccctggtt  10140
   aaaaacaagg tttataaata ttggtttaaa agcaggttaa aagacaggtt agcggtggcc  10200
   gaaaaacggg cggaaaccct tgcaaatgct ggattttctg cctgtggaca gcccctcaaa  10260
   tgtcaatagg tgcgcccctc atctgtcagc actctgcccc tcaagtgtca aggatcgcgc  10320
   ccctcatctg tcagtagtcg cgcccctcaa gtgtcaatac cgcagggcac ttatccccag  10380
   gcttgtccac atcatctgtg ggaaactcgc gtaaaatcag gcgttttcgc cgatttgcga  10440
   ggctggccag ctccacgtcg ccggccgaaa tcgagcctgc ccctcatctg tcaacgccgc  10500
   gccgggtgag tcggcccctc aagtgtcaac gtccgcccct catctgtcag tgagggccaa  10560
   gttttccgcg aggtatccac aacgccggcg gccggccgcg gtgtctcgca cacggcttcg  10620
   acggcgtttc tggcgcgttt gcagggccat agacggccgc cagcccagcg gcgagggcaa  10680
   ccagcccggt gagcgtcgga aagggtcgac atcttgctgc gttcggatat tttcgtggag  10740
   ttcccgccac agacccggat tgaaggcgag atccagcaac tcgcgccaga tcatcctgtg  10800
   acggaacttt ggcgcgtgat gactggccag gacgtcggcc gaaagagcga caagcagatc  10860
   acgattttcg acagcgtcgg atttgcgatc gaggattttt cggcgctgcg ctacgtccgc  10920
   gaccgcgttg agggatcaag ccacagcagc ccactcgacc ttctagccga cccagacgag  10980
   ccaagggatc tttttggaat gctgctccgt cgtcaggctt tccgacgttt gggtggttga  11040
   acagaagtca ttatcgtacg gaatgccagc actcccgagg ggaaccctgt ggttggcatg  11100
   cacatacaaa tggacgaacg gataaacctt ttcacgccct tttaaatatc cgttattcta  11160
   ataaacgctc ttttctctta ggtttacccg ccaatatatc ctgtcaaaca ctgatagttt  11220
   aaactgaagg cgggaaacga caatctgatc atgagcggag aattaaggga gtcacgttat  11280
   gacccccgcc gatgacgcgg gacaagccgt tttacgtttg gaactgacag aaccgcaacg  11340
   attgaaggag ccactcagcc ccaatacgca aaccgcctct ccccgcgcgt tggccgattc  11400
   attaatgcag ctggcacgac aggtttcccg actggaaagc gggcagtgag cgcaacgcaa  11460
   ttaatgtgag ttagctcact cattaggcac cccaggcttt acactttatg cttccggctc  11520
   gtatgttgtg tggaattgtg agcggataac aatttcacac aggaaacagc tatgaccatg  11580
   attacgccaa gctatttagg tgacactata gaatactcaa gctatgcatc caacgcgttg  11640
   ggagctctcc catatcgacc tgcaggcggc cgctcgacga attaattcca atcccacaaa  11700
   aatctgagct taacagcaca gttgctcctc tcagagcaga atcgggtatt caacaccctc  11760
   atatcaacta ctacgttgtg tataacggtc cacatgccgg tatatacgat gactggggtt  11820
   gtacaaaggc ggcaacaaac ggcgttcccg gagttgcaca caagaaattt gccactatta  11880
   cagaggcaag agcagcagct gacgcgtaca caacaagtca gcaaacagac aggttgaact  11940
   tcatccccaa aggagaagct caactcaagc ccaagagctt tgctaaggcc ctaacaagcc  12000
   caccaaagca aaaagcccac tggctcacgc taggaaccaa aaggcccagc agtgatccag  12060
   ccccaaaaga gatctccttt gccccggaga ttacaatgga cgatttcctc tatctttacg  12120
   atctaggaag gaagttcgaa ggtgaaggtg acgacactat gttcaccact gataatgaga  12180
   aggttagcct cttcaatttc agaaagaatg ctgacccaca gatggttaga gaggcctacg  12240
   cagcaggtct catcaagacg atctacccga gtaacaatct ccaggagatc aaataccttc  12300
   ccaagaaggt taaagatgca gtcaaaagat tcaggactaa ttgcatcaag aacacagaga  12360
   aagacatatt tctcaagatc agaagtacta ttccagtatg gacgattcaa ggcttgcttc  12420
   ataaaccaag gcaagtaata gagattggag tctctaaaaa ggtagttcct actgaatcta  12480
   aggccatgca tggagtctaa gattcaaatc gaggatctaa cagaactcgc cgtgaagact 12540
   ggcgaacagt tcatacagag tcttttacga ctcaatgaca agaagaaaat cttcgtcaac 12600
   atggtggagc acgacactct ggtctactcc aaaaatgtca aagatacagt ctcagaagac 12660
   caaagggcta ttgagacttt tcaacaaagg ataatttcgg gaaacctcct cggattccat 12720
   tgcccagcta tctgtcactt catcgaaagg acagtagaaa aggaaggtgg ctcctacaaa 12780
   tgccatcatt gcgataaagg aaaggctatc attcaagatc tctctgccga cagtggtccc 12840
   aaagatggac ccccacccac gaggagcatc gtggaaaaag aagacgttcc aaccacgtct 12900
   tcaaagcaag tggattgatg tgacatctcc actgacgtaa gggatgacgc acaatcccac 12960
   tatccttcgc aagacccttc ctctatataa ggaagttcat ttcatttgga gaggacacgc 13020
   tcgagacaag tttgtacaaa aaagctgaac gagaaacgta aaatgatata aatatcaata 13080
   tattaaatta gattttgcat aaaaaacaga ctacataata ctgtaaaaca caacatatcc 13140
   agtcactatg aatcaactac ttagatggta ttagtgacct gtagtcgacc gacagccttc 13200
   caaatgttct tcgggtgatg ctgccaactt agtcgaccga cagccttcca aatgttcttc 13260
   tcaaacggaa tcgtcgtatc cagcctactc gctattgtcc tcaatgccgt attaaatcat 13320
   aaaaagaaat aagaaaaaga ggtgcgagcc tcttttttgt gtgacaaaat aaaaacatct 13380
   acctattcat atacgctagt gtcatagtcc tgaaaatcat ctgcatcaag aacaatttca 13440
   caactcttat acttttctct tacaagtcgt tcggcttcat ctggattttc agcctctata 13500
   cttactaaac gtgataaagt ttctgtaatt tctactgtat cgacctgcag actggctgtg 13560
   tataagggag cctgacattt atattcccca gaacatcagg ttaatggcgt ttttgatgtc 13620
   attttcgcgg tggctgagat cagccacttc ttccccgata acggagaccg gcacactggc 13680
   catatcggtg gtcatcatgc gccagctttc atccccgata tgcaccaccg ggtaaagttc 13740
   acgggagact ttatctgaca gcagacgtgc actggccagg gggatcacca tccgtcgccc 13800
   gggcgtgtca ataatatcac tctgtacatc cacaaacaga cgataacggc tctctctttt 13860
   ataggtgtaa accttaaact gcatttcacc agtccctgtt ctcgtcagca aaagagccgt 13920
   tcatttcaat aaaccgggcg acctcagcca tcccttcctg attttccgct ttccagcgtt 13980
   cggcacgcag acgacgggct tcattctgca tggttgtgct taccagaccg gagatattga 14040
   catcatatat gccttgagca actgatagct gtcgctgtca actgtcactg taatacgctg 14100
   cttcatagca cacctctttt tgacatactt cgggtagtgc cgatcaacgt ctcattttcg 14160
   ccaaaagttg gcccagggct tcccggtatc aacagggaca ccaggattta tttattctgc 14220
   gaagtgatct tccgtcacag gtatttattc ggcgcaaagt gcgtcgggtg atgctgccaa 14280
   cttagtcgac tacaggtcac taataccatc taagtagttg attcatagtg actggatatg 14340
   ttgtgtttta cagtattatg tagtctgttt tttatgcaaa atctaattta atatattgat 14400
   atttatatca ttttacgttt ctcgttcagc tttcttgtac aaagtggtct cgaggaattc 14460
   ggtaccccag cttggtaagg aaataattat tttctttttt ccttttagta taaaatagtt 14520
   aagtgatgtt aattagtatg attataataa tatagttgtt ataattgtga aaaaataatt 14580
   tataaatata ttgtttacat aaacaacata gtaatgtaaa aaaatatgac aagtgatgtg 14640
   taagacgaag aagataaaag ttgagagtaa gtatattatt tttaatgaat ttgatcgaac 14700
   atgtaagatg atatactagc attaatattt gttttaatca taatagtaat tctagctggt 14760
   ttgatgaatt aaatatcaat gataaaatac tatagtaaaa ataagaataa ataaattaaa 14820
   ataatatttt tttatgatta atagtttatt atataattaa atatctatac cattactaaa 14880
   tattttagtt taaaagttaa taaatatttt gttagaaatt ccaatctgct tgtaatttat 14940
   caataaacaa aatattaaat aacaagctaa agtaacaaat aatatcaaac taatagaaac 15000
   agtaatctaa tgtaacaaaa cataatctaa tgctaatata acaaagcgca agatctatca 15060
   ttttatatag tattattttc aatcaacatt cttattaatt tctaaataat acttgtagtt 15120
   ttattaactt ctaaatggat tgactattaa ttaaatgaat tagtcgaaca tgaataaaca 15180
   aggtaacatg atagatcatg tcattgtgtt atcattgatc ttacatttgg attgattaca 15240
   gttgggaagc tgggttcgaa atcgataagc ttggatcctc tagaccactt tgtacaagaa 15300
   agctgaacga gaaacgtaaa atgatataaa tatcaatata ttaaattaga ttttgcataa 15360
   aaaacagact acataatact gtaaaacaca acatatccag tcactatgaa tcaactactt 15420
   agatggtatt agtgacctgt agtcgactaa gttggcagca tcacccgacg cactttgcgc 15480
   cgaataaata cctgtgacgg aagatcactt cgcagaataa ataaatcctg gtgtccctgt 15540
   tgataccggg aagccctggg ccaacttttg gcgaaaatga gacgttgatc ggatttcaca 15600
   actcttatac ttttctctta caagtcgttc ggcttcatct ggattttcag cctctatact 15660
   tactaaacgt gataaagttt ctgtaatttc tactgtatcg acctgcagac tggctgtgta 15720
   taagggagcc tgacatttat attccccaga acatcaggtt aatggcgttt ttgatgtcat 15780
   tttcgcggtg gctgagatca gccacttctt ccccgataac ggagaccggc acactggcca 15840
   tatcggtggt catcatgcgc cagctttcat ccccgatatg caccaccggg taaagttcac 15900
   gggagacttt atctgacagc agacgtgcac tggccagggg gatcaccatc cgtcgcccgg 15960
   gcgtgtcaat aatatcactc tgtacatcca caaacagacg ataacggctc tctcttttat 16020
   aggtgtaaac cttaaactgc atttcaccag tccctgttct cgtcagcaaa agagccgttc 16080
   atttcaataa accgggcgac ctcagccatc ccttcctgat tttccgcttt ccagcgttcg 16140
   gcacgcagac gacgggcttc attctgcatg gttgtgctta ccagaccgga gatattgaca 16200
   tcatatatgc cttgagcaac tgatagctgt cgctgtcaac tgtcactgta atacgctgct 16260
   tcatagcaca cctctttttg acatacttct gttcttgatg cagatgattt tcaggactat 16320
   gacactagcg tatatgaata ggtagatgtt tttattttgt cacacaaaaa agaggctcgc 16380
   acctcttttt cttatttctt tttatgattt aatacggcat tgaggacaat agcgagtagg 16440
   ctggatacga cgattccgtt tgagaagaac atttggaagg ctgtcggtcg actaagttgg 16500
   cagcatcacc cgaagaacat ttggaaggct gtcggtcgac tacaggtcac taataccatc 16560
   taagtagttg attcatagtg actggatatg ttgtgtttta cagtattatg tagtctgttt 16620
   tttatgcaaa atctaattta atatattgat atttatatca ttttacgttt ctcgttcagc 16680
   ttttttgtac aaacttgtct agagtcctgc tttaatgaga tatgcgagac gcctatgatc 16740
   gcatgatatt tgctttcaat tctgttgtgc acgttgtaaa aaacctgagc atgtgtagct 16800
   cagatcctta ccgccggttt cggttcattc taatgaatat atcacccgtt actatcgtat 16860
   ttttatgaat aatattctcc gttcaattta ctgattgtac cctactactt atatgtacaa 16920
   tattaaaatg aaaacaatat attgtgctga ataggtttat agcgacatct atgatagagc 16980
   gccacaataa caaacaattg cgttttatta ttacaaatcc aattttaaaa aaagcggcag 17040
   aaccggtcaa acctaaaaga ctgattacat aaatcttatt caaatttcaa aaggccccag 17100
   gggctagtat ctacgacaca ccgagcggcg aactaataac gttcactgaa gggaactccg 17160
   gttccccgcc ggcgcgcatg ggtgagattc cttgaagttg agtattggcc gtccgctcta 17220
   ccgaaagtta cgggcaccat tcaacccggt ccagcacggc ggccgggtaa ccgacttgct 17280
   gccccgagaa ttatgcagca tttttttggt gtatgtgggc cccaaatgaa gtgcaggtca 17340
   aaccttgaca gtgacgacaa atcgttgggc gggtccaggg cgaattttgc gacaacatgt 17400
   cgaggctcag caggacctgc aggcatgcaa gctagcttac tagtgatgca tattctatag 17460
   tgtcacctaa atctgc                                                 17476